(19) Europäisches Patentamt
European Patent Office
Office européen des brevets

(11) EP 1 327 436 B1

(12) # FASCICULE DE BREVET EUROPEEN

(45) Date de publication et mention de la délivrance du brevet:
**10.05.2006 Bulletin 2006/19**

(21) Numéro de dépôt: **02292985.5**

(22) Date de dépôt: **03.12.2002**

(51) Int Cl.:
***A61K 8/97*** (2006.01) ***A61K 8/49*** (2006.01)
***A61K 8/89*** (2006.01) ***A61K 8/896*** (2006.01)
***A61K 8/895*** (2006.01) ***A61K 8/897*** (2006.01)
***A61K 8/898*** (2006.01) ***A61Q 19/04*** (2006.01)

(54) **Emulsion pour la coloration de la peau contenant un extrait de Sorgho et une silicone organomodifiée**

Hautfärbe Emulsion enthaltend ein Sorghumextrakt und ein organomodifiziertes Silikon

Skin dyeing emulsion containing a sorghum extract and an organomodified silicone

(84) Etats contractants désignés:
**AT BE BG CH CY CZ DE DK EE ES FI FR GB GR IE IT LI LU MC NL PT SE SI SK TR**

(30) Priorité: **10.01.2002 FR 0200254**

(43) Date de publication de la demande:
**16.07.2003 Bulletin 2003/29**

(73) Titulaire: **L'OREAL**
**75008 Paris (FR)**

(72) Inventeurs:
- **Seyler, Nathalie**
  **94700 Maisons-Alfort (FR)**
- **Elguidj, Irène**
  **92200 Neuilly (FR)**
- **Candau, Didier**
  **91570 Bievres (FR)**

(74) Mandataire: **Miszputen, Laurent**
**L'OREAL - D.I.P.I.**
**25-29 Quai Aulagnier**
**92600 Asnières (FR)**

(56) Documents cités:
**EP-A- 1 230 914** **FR-A- 2 757 383**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 327 436 B1

Printed by Jouve, 75001 PARIS (FR)

## Description

La présente invention a pour objet une émulsion cosmétique ou dermatologique, caractérisée par le fait qu'elle comporte :

- au moins une phase aqueuse et
- au moins une phase grasse .;
- au moins un extrait de sorgho ;
- au moins une silicone organomodifiée ; ladite composition ne contenant pas de sel de flavylium non substitué en position 3 et substitué par au moins un radical hydroxyle ou alcoxy.

L'invention concerne également ses utilisations pour la fabrication de compositions cosmétiques ou dermatologiques pour la coloration de la peau.

De nos jours, il est important d'avoir bonne mine et une peau bronzée est toujours signe de bonne santé. Cependant, le bronzage naturel n'est pas toujours souhaitable dans la mesure où il nécessite des expositions prolongées aux rayonnements UV, en particulier aux rayonnements UV-A qui provoquent le brunissement de la peau mais en contrepartie sont susceptibles d'induire une altération de celle-ci, notamment dans le cas d'une peau sensible ou d'une peau continuellement exposée au rayonnement solaire. Il est donc souhaitable de trouver une alternative au bronzage naturel qui soit compatible avec les exigences de telles peaux.

La plupart des produits cosmétiques destinés au bronzage artificiel de la peau sont à base de dérivés carbonylés permettant, par interaction avec les acides aminés de la peau, la formation de produits colorés.

A cet effet, on sait que la dihydroxyacétone, ou DHA, est un produit particulièrement intéressant qui est couramment utilisé en cosmétique comme agent de bronzage artificiel de la peau; appliqué sur cette dernière, notamment sur le visage, il permet d'obtenir un effet de bronzage ou de brunissage d'apparence semblable à celui qui peut résulter d'une exposition prolongée au soleil (bronzage naturel) ou sous une lampe UV.

Un inconvénient de la DHA est la lenteur avec laquelle la coloration se développe : il faut en effet compter plusieurs heures (3 à 5 heures en général) pour que soit révélée la coloration. Il existe donc une demande croissante de produits autobronzants agissant rapidement et conférant une coloration plus proche du bronzage naturel.

Ainsi, on est toujours à la recherche de nouveaux composés et de nouvelles compositions permettant de conférer artificiellement à la peau une coloration proche du bronzage naturel d'une manière simple, efficace, rapide et sans risque.

Les extraits de sorgho sont connus depuis longtemps comme colorants alimentaires. Ils procurent une teinte marron-rouge et présentent dans leur composition des flavonoïdes, des anthocyanidines et des tannins.

Or, à la suite d'importantes recherches menées dans le domaine de la coloration artificielle de la peau, la Demanderesse a maintenant découvert que des émulsions particulières contenant au moins une silicone organomodifiée et à titre d'agent de coloration de la peau au moins un extrait de sorgho, en l'absence de sel de flavylium non substitué en position 3 et substitué par au moins un radical hydroxyle ou alcoxy, permettaient non seulement de conférer immédiatement après l'application sur la peau du produit une coloration artificielle proche du bronzage naturel mais aussi présentaient une bonne stabilité ainsi qu'un bon agrément cosmétique.

Ces découvertes sont à l'origine de la présente invention.

La présente invention a donc pour objet une émulsion cosmétique ou dermatologique, caractérisée par le fait qu'elle comporte :

(a) au moins une phase aqueuse et

(b) au moins une phase grasse ;

(c) au moins un extrait de sorgho ;

(d) au moins une silicone organomodifiée; ladite composition ne contenant pas de sel de flavylium non substitué en position 3 et substitué par au moins un radical hydroxyle ou alcoxy.

Par émulsion cosmétique ou dermatologique, au sens de la présente invention et dans le texte qui suit, on entend toute émulsion dont la phase aqueuse et la phase grasse sont constituées de substances cosmétiquement ou dermatologiquement acceptables pour une application topique sur la peau.

La présente invention a également pour objet un procédé de coloration artificielle proche du bronzage naturel de la peau, caractérisé en ce qu'il consiste à appliquer sur celle-ci une quantité efficace d'une composition cosmétique telle que définie précédemment.

La présente invention a également pour objet l'utilisation de l'émulsion pour la fabrication de compositions cosmétiques pour la coloration de la peau.

La présente invention a également pour objet l'utilisation d'au moins un extrait de sorgho et d'une silicone organomodifiée dans une émulsion cosmétique ne contenant pas de sel de flavylium non substitué en position 3 et substitué par au moins un radical hydroxyle ou alcoxy, dans le but d'obtenir une coloration artificielle de la peau proche du bronzage naturel.

Les compositions et les utilisations conformes à l'invention permettent d'obtenir une coloration artificielle proche du bronzage naturel en un laps de temps court. Ainsi, on obtient une coloration immédiate qui permet une visualisation de l'application et par conséquent une meilleure homogénéité dans l'étalement de la composition sur la peau et donc de la coloration qui en résulte.

Au sens de la présente invention, on entendra, par « composition destinée à la coloration artificielle de la peau », une formulation ayant une affinité particulière pour la peau lui permettant de conférer à cette dernière une coloration durable, non-couvrante (à savoir n'ayant pas tendance à opacifier la peau) et qui ne s'élimine ni à l'eau ni à l'aide d'un solvant, et qui résiste à la fois au frottement et au lavage par une solution contenant des tensioactifs. Une telle coloration durable se distingue donc de la coloration superficielle et momentanée apportée par exemple par un produit de maquillage.

On entend par silicone, en conformité avec l'acception générale, tous polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyles notamment en $C_1$-$C_{10}$ et en particulier méthyle, les radicaux fluoroalkyles, les radicaux aryles et en particulier phényle. Les silicones sont définies plus en détail dans l'ouvrage de Walter NOLL "Chemistry and Technology of Silicones" (1968) Académie Press.

D'autres caractéristiques, aspects et avantages de la présente invention apparaîtront à la lecture de la description détaillée qui va suivre.

Les silicones organomodifiées utilisables conformément à l'invention sont des silicones telles que définies précédemment et comportant dans leur structure un ou plusieurs groupements organofonctionnels fixés par l'intermédiaire d'un radical hydrocarboné. Les silicones utilisables conformément à l'invention peuvent se présenter sous forme d'huiles, de cires, de résines ou de gommes. Elles peuvent être hydrosolubles ou insolubles dans l'eau.

Parmi les silicones organomodifiées, on peut citer les polyorganosiloxanes comportant :

(1) des groupements oxyalkylénés (en particulier oxyéthylénés et/ou oxypropylénés) telles que celles décrites dans la demande de brevet EP-0796615 et répondant aux formules suivantes :

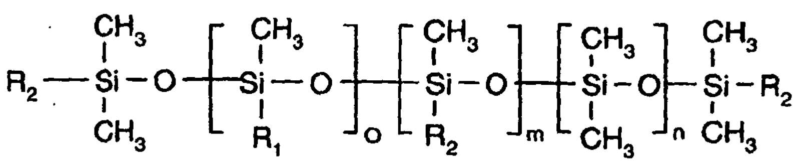 (I)

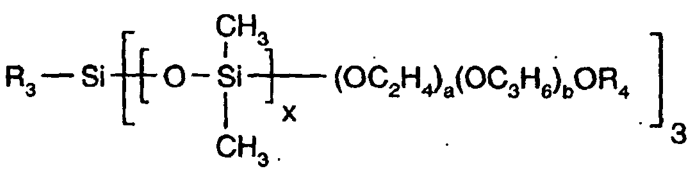 (II)

- $R_1$, identique ou différent, représente un radical alkyle, linéaire ou ramifié, en $C_1$-$C_{30}$ ou phényle.
- $R_2$, identique ou différent, $-C_cH_{2c}-(-O-C_2H_4)_a-(-O-C_3H_6)_b-(O-C_4H_8)_d-R_5$
- $R_3$, $R_4$, identiques ou différents, désignent un radical alkyle, linéaire ou ramifié, en $C_{1-}C_{12}$, et de préférence le radical méthyle,
- $R_5$, identique ou différent, est choisi parmi un atome d'hydrogène, un radical hydroxyle, un radical alkyle linéaire ou ramifié de 1 à 12 atomes de carbone, un radical alcoxy linéaire ou ramifié de 1 à 6 atomes de carbone, un radical acyloxy linéaire ou ramifié de 2 à 12 atomes de carbone, $NHCH_2CH_2COOM$, aminoalkyle éventuellement

substitué sur l'amine, carboxyacyle en $C_1$-$C_{30}$, un groupement phosphono éventuellement substitué,

$$-O-CO-(CH_2)_d-CO_2M,\ -NHCO(CH_2)_dOH,\ -NH_3Y.$$

- M, identique ou différent, désigne un atome d'hydrogène, Na, K, Li, $NH_4$ ou une amine organique,
- a varie de 0 à 50,
- b varie de 0 à 50,
- c varie de 0 à 4,
- a + b est supérieur ou égal à 1,
- d varie de 0 à 10,
- m varie de 0 à 20,
- n varie de 0 à 500,
- p varie de 0 à 20,
- x varie de 1 à 100,
- Y représente un anion minéral ou organique monovalent tel que halogénure (chlorure, bromure), sulfate, carboxylate (acétate, lactate, citrate) ; comme par exemple celles vendues sous les dénominations commerciales FLUID DC 193 par la société DOW CORNING, SILWET L 77 par la société OSI et MAZIL 756 par la société MAZER PPG ; on peut citer également les produits commercialisés sous les noms « Silicone DC 3225C » « DC Q2-5200 » par la société Dow Corning ;

(2) des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;

(3) des groupements anioniques du type 2-hydroxyalkylsulfonate ; 2-hydroxyalkylthiosulfate tels que les produits commercialisés par la société GOLDSCHMIDT sous les dénominations "ABIL S201" et "ABIL S255" ;

(4) des groupements thiols comme les produits commercialisés sous les dénominations "GP 72 A" et "GP 71 " de GENESEE ;

(5) des groupements anioniques du type carboxylique telles que les silicones organomodifiées décrites dans les demande WO95/23579 et EP-A-0 219 830, WO98/20883 et notamment celles répondant à la formule (III) suivante :

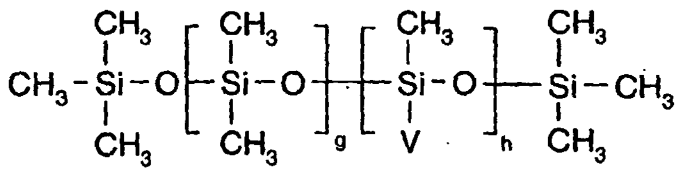

(III)

dans laquelle V est un radical -$(R^1O)_e$-$R^2$-$(OR^3)_f$-COOM, dans lequel $R^1$ et $R^3$ désignent indépendamment un radical alkylène linéaire ou ramifié ayant de 2 à 20 atomes de carbone, $R^2$ désigne un radical alkylène linéaire ou ramifié ayant de 1 à 50 atomes de carbone pouvant comprendre un groupement hydroxyle ; e représente 0 ou 1 et f est un nombre allant de 0 à 200 ; M désigne hydrogène, un métal alcalin ou alcalino-terreux, $NH_4$, un groupement ammonium quaternaire tel qu'un groupement mono-, di-, tri- ou tétra (alkyl $C_{1-}C_4$) ammonium ; g et h sont des nombres allant de 0 à 1000, la somme c+d allant de préférence de 2 à 1000 ;par exemple celles commercialisés sous la dénomination Huile M 642 par la société WACKER, sous les dénominations SLM 23 000/1, SLM 23 000/2 par la société WACKER, sous la dénomination 176-12057 par la société GENERAL ELECTRIC, sous la dénomination FZ 3703 par la société OSI, sous la dénomination BY 16 880 par la société TORAY SILICONE ; on peut citer également les produits décrits dans le brevet EP 186 507 de la société CHISSO CORPORATION, ou de type alkylcarboxyliques comme ceux présents dans le produit X-22-3701 E de la société SHIN-ETSU ;

(6) des groupements hydroxylés comme les polyorganosiloxanes à fonction hydroxyalkyle décrits dans la demande de brevet français FR-A-85 16334 répondant à la formule (IV) :

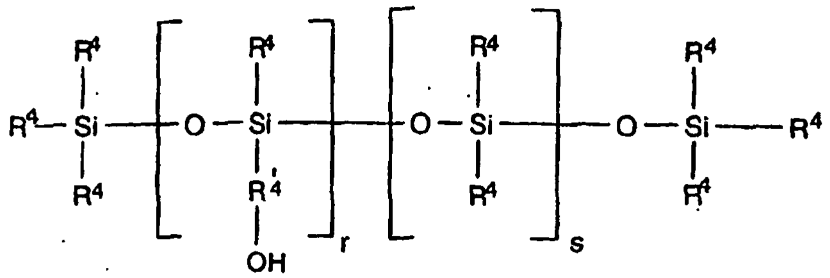

(IV)

dans laquelle les radicaux $R^4$ identiques ou différents sont choisis parmi les radicaux méthyle et phényle ; au moins 60 % en mole des radicaux $R^4$ désignant méthyle ; le radical $R'^4$ est un chaînon alkylène divalent hydrocarboné en $C_2$-$C_{18}$ ; r est compris entre 1 et 30 inclus ; s est compris entre 1 et 150 inclus ;

(7) des groupements acyloxyalkyle tels que par exemple les polyorganosiloxanes décrits dans la demande de brevet français FR-A-2641185 et répondant à la formule (V) :

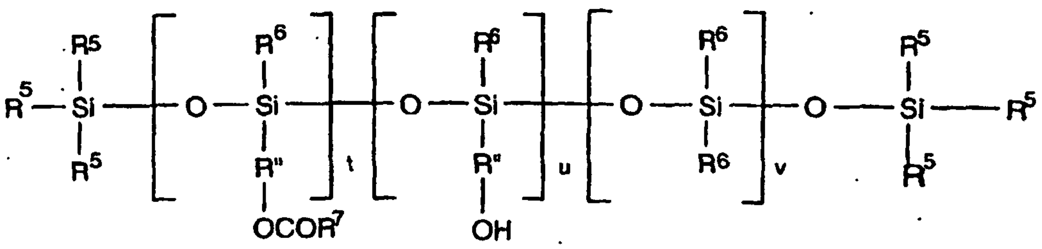

(V)

dans laquelle :

$R^5$ désigne un groupement méthyle, phényle, $-OCOR^7$, hydroxyle, un seul des radicaux $R^5$ par atome de silicium pouvant être OH ;
$R^6$ désigne méthyle, phényle ; au moins 60 % en proportion molaire de l'ensemble des radicaux $R^5$ et $R^6$ désignant méthyle;
$R^7$ désigne alkyle ou alcényle en $C_8$-$C_{20}$ ;
R" désigne un radical alkylène hydrocarboné divalent, linéaire ou ramifié en $C_2$-$C_{18}$ ;
t est compris entre 1 et 120 inclus ;
u est compris entre 1 et 30 ;
v est égal à 0 ou est inférieur à 0,5 t, t + u étant compris entre 1 et 30 ; les polyorganosiloxanes de formule (V) peuvent contenir des groupements :

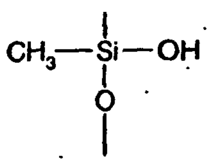

dans des proportions ne dépassant pas 15 % de la somme t + u + v ;

(8) des groupements aminés substitués ou non telles que les silicones décrites dans la demande de brevet EP-A-0852 488 et notamment les silicones aminées choisies parmi :

(a) les polysiloxanes répondant à la formule :

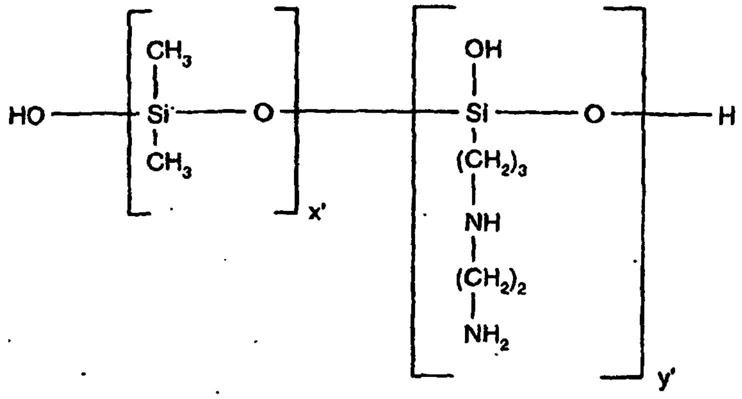 (VI)

dans laquelle x' et y' sont des nombres entiers dépendant du poids moléculaire, généralement tels que ledit poids moléculaire en nombre est compris entre 5 000 et 500 000;

(b) les polymères cationiques siliconés répondant à la formule :

$$R^8{}_iG_{3-i}\text{-}Si(OSiG_2)_k\text{-}(OSiG_jR^8{}_{2-j})_l\text{-}O\text{-}SiG_{3-i}\text{-}R^8{}_i \quad \text{(VII)}$$

dans laquelle :

G est un atome d'hydrogène, ou un groupement phényle, OH, ou alkyle en $C_1$-$C_8$, par exemple méthyle,
i désigne le nombre 0 ou un nombre entier de 1 à 3, en particulier 0,
j désigne 0 ou 1, et en particulier 1,
k et l sont des nombres tels que la somme (k+l) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
$R^8$ est un radical monovalent de formule $-C_qH_{2q}L$ dans laquelle q est un nombre de 2 à 8 et L est un groupement aminé éventuellement quaternisé choisi parmi les groupements :

$$-NR^9\text{-}CH_2\text{-}CH_2\text{-}N(R^9)_2$$

$$-N(R^9)_2$$

$$-N^{\oplus}(R^9)_3A^-$$

$$-N(R^9)\text{-}CH_2\text{-}CH_2\text{-}N^{\oplus}R^9H_2\ A^-,$$

dans lesquels $R^9$ désigne hydrogène, phényle, benzyle, ou un radical hydrocarboné saturé monovalent, par exemple un radical alkyle ayant de 1 à 20 atomes de carbone et $A^-$ représente un ion halogénure tel que par exemple fluorure, chlorure, bromure ou iodure.

(c) les polymères cationiques siliconés répondant à la formule :

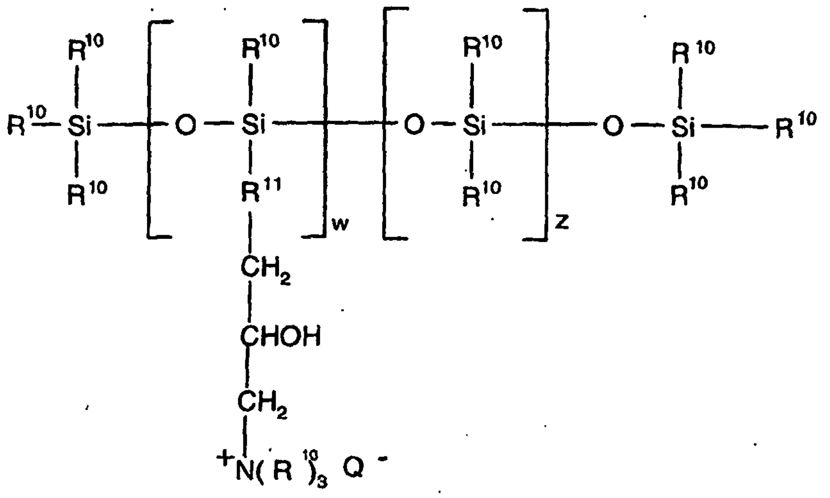 (VIII)

dans laquelle :

$R^{10}$ représente un radical hydrocarboné monovalent ayant de 1 à 18 atomes de carbone, et en particulier un radical alkyle en $C_1$-$C_{18}$, ou alcényle en $C_2$-$C_{18}$, par exemple méthyle; $R^{11}$ représente un radical hydrocarboné divalent, notamment un radical alkylène en $C_1$-$C_{18}$ ou un radical alkylèneoxy divalent en $C_1$-$C_{18}$, par exemple en $C_1$-$C_8$ ;
$Q^-$ est un ion halogénure, notamment chlorure ;
w représente une valeur statistique moyenne de 2 à 20 et en particulier de 2 à 8 ;
z représente une valeur statistique moyenne de 20 à 200 et en particulier de 20 à 50 ; à titre d'exemple de silicones aminées, on peut citer les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING.;

(9) des groupements hydroxyacylamino, comme les polyorganosiloxanes décrits dans la demande EP 342 834. On peut citer par exemple le produit Q2-8413 de la société DOW CORNING.

(10) des groupements de type aryle éventuellement substitué comme par exemple phényle, naphtyle, benzyle ou phénéthyle telles que les silicones arylées non-volatiles décrites dans la demande de brevet EP-A-822202 ; à titre d'exemples de ces composés, on peut citer ceux vendus par la société BAYER sous le nom Huile BAYSILONE FLUID PD5, par la société DOW CORNING sous le nom DOW CORNING 556 FLUID, par RHONE POULENC sous les noms MIRASIL DPDM, RHODORSIL HUILE 510 V 100, RHODORSIL HUILE 550, RHODORSIL HUILE 510V500, RHODORSIL HUILE 710, sous les dénomination WACKER BELSIL PDM 20, PDM 200, PDM 1000 par la société WACKER.

La ou les silicones organomodifiées selon la présente invention sont de préférence présentes dans des concentrations allant de 0,1 à 40 % par rapport au poids total de la composition, et plus préférentiellement en une quantité allant de 0,5 à 20 %.

Les compositions conformes à la présente invention conduisent en général au bout de 30 minutes après application sur une peau claire à raison de $2mg/cm^2$ à un assombrissement caractérisé dans le système de mesure colorimétrique (L*, a*, b*) par un $\Delta L^*$ allant de -0,5 à - 20. De façon plus préférentielle, $\Delta L^*$ variera de - 0,5 à - 15.

Les compositions conformes à la présente invention apportent au bout de 30 minutes après application sur la peau à raison de $2mg/cm^2$ une coloration sur une peau claire qui est définie dans le système de mesure colorimétrique (L*, a*, b*), par un rapport $\Delta a^*/\Delta b^*$ allant de 0,5 à 3 et encore plus particulièrement de 0, 8 à 2.

Selon la présente invention, on entend « par peau claire », des peaux non bronzées dont on peut définir les caractéristiques colorimétriques par leur angle ITA tel que défini dans la publication de A. Chardon et al. « Skin Colour Typology and Suntanning Pathways » et présentée au $16^{th}$ IFSCC congress oct 8-10 1990 de New-York, et in *Int. J. Cosm. Sci.* **13** 191-208 (1991). Les peaux claires telles que définies dans cette classification ont un angle ITA compris entre 35 et 55.

Dans le système de mesure colorimétrique (L*, a*, b*) :

L* représente la luminance ou clarté, a* représente l'axe rouge-vert (-a*= vert, +a*= rouge) et b* représente l'axe jaune-bleu (-b*=bleu, +b*=jaune). Ainsi, a* et b* expriment la nuance de la peau.

ΔL* traduit l'assombrissement de la couleur : plus le ΔL* est négatif, plus la couleur s'est assombrie avec:

$$\Delta L^* = L^* \text{ peau non colorée} - L^* \text{ peau colorée}$$

Le rapport Δa*/Δb* traduit l'équilibre rouge/jaune et donc la nuance avec :

$$\Delta a^* = a^* \text{ peau non colorée} - a^* \text{ peau colorée}$$

$$\Delta b^* = b^* \text{ peau non colorée} - b^* \text{ peau colorée}$$

Les extraits de sorgho conformes à l'invention sont obtenus à partir de la plante entière, des tiges, des graines ou des feuilles du genre Sorghum. Les espèces préférées du Sorghum sont choisies parmi le Sorghum bicolor, le Sorghum caudatum, le Sorghum nervosum, le Sorghum durra, le Sorghum vulgare et les Sorghum en association avec du Colletotrichum Graminicola.

Les extraits de sorgho conformes à l'invention sont plus particulièrement les extraits de *Sorghum vulgare* tels que le produit commercial Sorghum Extract Absorbance >30 vendu par la société Premier Specialties .

Les extraits de sorgho conformes à l'invention sont issus de l'extraction de la plante entière ou des parties de plantes citées ci-dessus qui peuvent être à l'état frais ou à l'état sec.

Les extraits de sorgho conformes à l'invention peuvent être obtenus selon un procédé comprenant les étapes suivantes :

(a) une extraction de la plante entière, des tiges, des graines ou des feuilles de Sorghum dans un milieu aqueux pouvant contenir en plus au moins un solvant organique ;
(b) une macération dans un milieu alcalin présentant un pH de l'ordre 11-12.
(c) éventuellement une précipitation du milieu de macération par addition d'un acide de telle sorte à atteindre un pH de l'ordre de 1-2.

L''extraction peut être réalisée en milieu acide comme décrit dans les brevets chinois CN 1035512 C et CN 1064284A et dans la publication de M KOUDA-BONAFOS, E CZYZEWSKA, M NACRO et AC OEHLSCHLAGER. « Isolation of apigenin from leaf sheats of Sorghum caudatum » Journal of Chemical ecology, Vol. 20, N°8, p2123-2125 (1995).

Elle peut également être réalisée en milieu alcalin suivi d'une précipitation en milieu acide comme décrit dans le brevet chinois CN 1065079A et dans la publication de JP REY, JL POUSSET, J LEVESQUE et P WANTY. « isolation and composition of a natural dye from the stems of sorghum bicolor (L.) Moench subsp. Americanum caudatum » Cereal Chem. Vol 70(6), p759-760 (1993).

L'extraction peut aussi être réalisée en milieu organique comme décrit dans les publications de J WANG « Studies on extraction of pigment from sorghum husks and its properties » Huaxue Shijie, Vol 39 (4), p211-213 (1998) et de A SEREME, M KOUDA-BONAFOS et M NACRO « Phenolic compounds in Sorghum caudatum tissues during plant development » Biomass and Bioenergy Vol 4(1), p69-71 (1993).

Les solvants organiques utilisés pour l'extraction peuvent être des alcools comme l'éthanol, le méthanol, l'alcool propylique normal primaire, l'alcool isopropylique, l'alcool butylique normal primaire, le propylène glycol et le glycérol par exemple. Les solvants organiques peuvent être également représenté par l'éther diéthylique, l'acétone, l'éthylméthylcétone, l'acétate d'éthyle par exemple. Les solvants organiques utilisés peuvent également être des fluides supercritiques ou des solvants fluorées comme le dodécafluoropentane, le tétradécafluorohexane, la N-méthyl morpholyne perfluoré et le méthoxy nonafluorobutane par exemple.

L'étape (b) de macération en milieu alcalin peut être réalisée pendant une période de 15-25 jours à une température de 60-80°C dans une solution d'hydroxyde de sodium 0.1N présentant un pH de l'ordre de 11-12.

L'étape (c) de précipitation par addition d'acide peut être effectuée par exemple avec de l'acide chlorhydrique

10N pour atteindre un pH de l'ordre de 1-2. La suspension aqueuse ainsi obtenue est ensuite filtrée pour récupérer le précipité qui est ensuite séché.

Les étapes (b) et (c) peuvent être répétées plusieurs fois.

La concentration en extrait de Sorgho conforme à l'invention varie de préférence de 0,0001 à 10%, et encore plus préférentiellement de 0,001 à 5% en poids, par rapport au poids total de la composition.

Les compositions de l'invention peuvent être préparées selon les techniques bien connues de l'homme de l'art, en particulier celles destinées à la préparation d'émulsions de type huile-dans-eau ou eau-dans-huile.

Ces compositions peuvent se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

De façon particulièrement préférée, les compositions selon l'invention se présentent sous forme d'émulsion eau-dans-silicone et la silicone organomodifiée utilisée comporte de préférence des groupements oxyalkylénés (en particulier oxyéthylénés et/ou oxypropylénés) comme celles de formule (I) ou (II) définies précedemment.

Les compositions selon l'invention peuvent également contenir des agents de bronzage et/ou de brunissage artificiels de la peau (agents autobronzants).

Les agents autobronzants mono ou polycarbonylés sont choisis par exemple parmi l'isatine, l'alloxane, la ninhydrine, le glycéraldéhyde, l'aldéhyde mésotartrique, la glutaraldéhyde, l'érythrulose, les dérivés de pyrazolin-4,5-diones telles que décrits dans la demande de brevet FR 2466492 et WO 9735842, la dihydroxyacétone (DHA), les dérivés de 4,4-dihydroxypyrazolin-5-ones telles que décrits dans la demande de brevet EP 903342, ces agents autobronzants pouvant être associés ou non à des colorants directs ou des dérivés indoliques.

Dans un mode de réalisation préféré de l'invention on utilisera plus particulièrement la dihydroxyacétone (DHA).

Les agents autobronzants mono ou polycarbonylés sont généralement présents dans les compositions selon l'invention dans des proportions allant de 0,1 à 10% en poids par rapport au poids total de la composition, et de préférence de 0,2 à 8% en poids par rapport au poids total de la composition

Selon une forme particulière, les compositions de l'invention peuvent contenir en plus un ou plusieurs agents filtrant le rayonnement ultra violet.

Les agents filtrant le rayonnement ultra violet peuvent être choisis parmi les filtres UV organiques ou les agents filtrant les radiations UV minéraux.

Les filtres UV organiques conformes à l'invention peuvent être hydrosolubles, liposolubles ou insolubles dans les solvants usuels cosmétiques. Ils sont choisis notamment parmi les anthranilates; les dérivés cinnamiques; les dérivés de dibenzoylméthane; les dérivés salicyliques, les dérivés du camphre ; les dérivés de triazine tels que ceux décrits dans les demandes de brevet US 4367390, EP863145, EP517104, EP570838, EP796851, EP775698, EP878469 et EP933376 ; les dérivés de la benzophénone notamment ceux décrits dans les demandes EP-A-1046391 et DE10012408 ; les dérivés de benzalmalonate, les dérivés de $\beta,\beta'$-diphénylacrylate, les dérivés de benzotriazole, les dérivés de benzimidazole ; les imadazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US5,166,355, GB2303549, DE19726184 et EP893119 ; les polymères filtres et silicones filtres tels que ceux décrits notamment dans la demande WO93/04665 ; les dimères dérivés d'$\alpha$-alkylstyrène tels que ceux décrits dans la demande de brevet DE19855649 ; les dérivés de 4,4-diarylbutadiène tels que ceux décrits dans les demandes de brevet EP0967200, DE19755649, EP1333981 et leurs mélanges.

Comme exemples de filtres organiques, on peut citer désignés ci-dessus sous leur nom INCI :

<u>Dérivés de l'acide para-aminobenzoique :</u>

- PABA,
- Ethyl PABA,
- Ethyl Dihydroxypropyl PABA,
- Ethylhexyl Diméthyl PABA vendu notamment sous le nom « ESCALOL 507 » par ISP,
- Glyceryl PABA,
- PEG-25 PABA vendu sous le nom « UVINUL P25 » par BASF,

<u>Dérivés salicyliques :</u>

- Homosalate vendu sous le nom « EUSOLEX HMS » par RONA/EM INDUSTRIES,

- Ethylhexyl Salicylate vendu sous le nom « NEO HELIOPAN OS » par HAARMANN et REIMER,
- Dipropyleneglycol Salicylate vendu sous le nom « DIPSAL » par SCHER,
- TEA Salicylate, vendu sous le nom « NEO HELIOPAN TS » par HAARMANN et REIMER,

Dérivés du dibenzoylméthane :

- Butyl Methoxydibenzoylmethane vendu notamment sous le nom commercial « PARSOL 1789 » par HOFFMANN LA ROCHE,
- Isopropyl Dibenzoylmethane,

Dérivés cinnamiques :

- Ethylhexyl Methoxycinnamate vendu notamment sous le nom commercial « PARSOL MCX » par HOFFMANN LA ROCHE,
- Isopropyl Methoxy cinnamate,
- Isoamyl Methoxy cinnamate vendu sous le nom commercial « NEO HELIOPAN E 1000 » par HAARMANN et REIMER,
- Cinoxate,
- DEA Methoxycinnamate,
- - Diisopropyl Methylcinnamate,
- Glyceryl Ethylhexanoate Dimethoxycinnamate

Dérivés de β,β'-diphénylacrylate :

- Octocrylene vendu notamment sous le nom commercial « UVINUL N539 » par BASF,
- Etocrylene, vendu notamment sous le nom commercial « UVINUL N35 » par BASF,

Dérivés de la benzophénone :

- Benzophenone-1 vendu sous le nom commercial « UVINUL 400 » par BASF,
- Benzophenone-2 vendu sous le nom commercial « UVINUL D50 » par BASF
- Benzophenone-3 ou Oxybenzone, vendu sous le nom commercial « UVINUL M40 » par BASF,
- Benzophenone-4 vendu sous le nom commercial « UVINUL MS40 » par BASF,
- Benzophenone-5
- Benzophenone-6 vendu sous le nom commercial « HELISORB 11 » par NORQUAY
- Benzophenone-8 vendu sous le nom commercial « SPECTRA-SORB UV-24 » PAR AMERICAN CYANAMID
- Benzophenone-9 vendu sous le nom commercial« UVINUL DS-49» par BASF,
- Benzophenone-12

Dérivés du benzylidène camphre :

- 3-Benzylidene camphor fabriqué sous le nom « MEXORYL SD» par CHIMEX,
- 4-Methylbenzylidene camphor vendu sous le nom « EUSOLEX 6300 » par MERCK,
- Benzylidene Camphor Sulfonic Acid fabriqué sous le nom « MEXORYL SL» par CHIMEX,
- Camphor Benzalkonium Methosulfate fabriqué sous le nom « MEXORYL SO » par CHIMEX,
- -Terephthalylidene Dicamphor Sulfonic Acid fabriqué sous le nom « MEXORYL SX » par CHIMEX,
- Polyacrylamidomethyl Benzylidene Camphor fabriqué sous le nom « MEXORYL SW » par CHIMEX,

Dérivés du phenyl benzimidazole :

- Phenylbenzimidazole Sulfonic Acid vendu notamment sous le nom commercial « EUSOLEX 232 » par MERCK,
- Benzimidazilate vendu sous le nom commercial commercial « NEO HELIOPAN AP » par HAARMANN et REIMER,

Dérivés de la triazine :

- Anisotriazine vendu sous le nom commercial «TINOSORB S » par CIBA GEIGY,
- Ethylhexyl triazone vendu notamment sous le nom commercial «UVINUL T150 » par BASF,
- Diethylhexyl Butamido Triazone vendu sous le nom commercial « UVASORB HEB » par SIGMA 3V,
- la 2,4,6-tris-(4'-amino benzalmalonate de diisobutyle)-s- triazine.

Dérivés du phenyl benzotriazole :

- Drometrizole Trisiloxane vendu sous le nom « SILATRIZOLE » par RHODIA CHIMIE ,
- Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

Dérivés anthraniliques :

- Menthyl anthranilate vendu sous le nom commercial commercial « NEO HELIOPAN MA » par HAARMANN et REIMER,

Dérivés d'imidazolines :

- Ethylhexyl Dimethoxybenzylidene Dioxoimidazoline Propionate,

Dérivés du benzalmalonate :

- Polyorganosiloxane à fonctions benzalmalonate vendu sous la dénomination commerciale « PARSOL SLX » par HOFFMANN LA ROCHE

Dérivés de 4,4-diarylbutadiène :

- 1,1-dicarboxy (2,2'-diméthyl-propyl)-4,4-diphénylbutadiène

et leurs mélanges.

Les filtres UV organiques plus particulièrement préférés sont choisis parmi les composés suivants :

- Ethylhexyl Salicylate,
- Butyl Methoxydibenzoylmethane,
- Ethylhexyl Methoxycinnamate,
- Octocrylene,

- Phenylbenzimidazole Sulfonic Acid,
- Terephthalylidene Dicamphor Sulfonic,
- Benzophenone-3,
- Benzophenone-4,
- Benzophenone-5,
- 4-Methylbenzylidene camphor,
- Benzimidazilate,
- Anisotriazine,
- Ethylhexyl triazone,
- Diethylhexyl Butamido Triazone,
- Methylene bis-Benzotriazolyl Tetramethylbutylphenol, 1,1-dicarboxy (2,2'-diméthyl-propyl)-4,4-diphénylbutadiène
- Drometrizole Trisiloxane,

et leurs mélanges.

Les agents filtrant minéraux sont généralement des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les agents filtrants les radiations conformes à l'invention sont généralement présents dans les compositions selon l'invention dans des proportions allant de 0,1 à 20% en poids par rapport au poids total de la composition, et de préférence allant de 0,2 à 15% en poids par rapport au poids total de la composition.

Les compositions de l'invention peuvent comprendre en outre des adjuvants cosmétiques classiques notamment choisis parmi les corps gras, les solvants organiques, les épaississants, les adoucissants, les opacifiants, les stabilisants, les émollients, les agents anti-mousse, les agents hydratants, les parfums, les conservateurs, les polymères, les charges, les séquestrants, les propulseurs, les agents alcalinisants ou acidifiants ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de paraffine, les huiles de silicone, volatiles ou non, les isoparaffines, les polyoléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs. Ces derniers peuvent être choisis parmi les glycols et les éthers de glycol comme l'éthylène glycol, le propylène glycol, le butylène glycol, le dipropylène glycol ou le diéthylène glycol.

Bien entendu, l'homme de l'art veillera à choisir ce ou ces éventuels composés complémentaires et/ou leurs quantités de manière telle que les propriétés avantageuses, en particulier la rémanence à l'eau, la stabilité, attachées intrinsèquement aux émulsions conformes à l'invention ne soient pas, ou substantiellement pas, altérées par la ou les adjonctions envisagées.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR2315991 et FR2416008).

Lorsque la composition cosmétique selon l'invention est utilisée pour la coloration de l'épiderme humain, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de poudre, de stick, de mousse aérosol ou de spray.

Comme indiqué en début de description, un autre objet de la présente invention réside dans l'utilisation d'une émulsion selon l'invention pour la fabrication de compositions cosmétiques pour la coloration de la peau et/ou des cheveux.

Des exemples concrets, mais nullement limitatifs, illustrant l'invention, vont maintenant être donnés.

**EXEMPLE 1 :**

Cet exemple vise à montrer dans un premier temps la tenue au lavage d'une composition A selon l'invention contenant l'extrait de sorgho dans un support émulsion (eau-dans-silicone) contenant une silicone organomodifiée par

rapport à une composition B contenant l'extrait de sorgho dans un support émulsion (huile-dans-eau) ne contenant pas de silicone organomodifiée.

Cet exemple vise à montrer, dans un deuxième temps, que la composition A conforme à la présente invention appliquée sur une peau claire conduit rapidement à une coloration proche d'un bronzage naturel contrairement à une émulsion C de l'art antérieur contenant une silicone organomodifiée mais contenant la DHA à la place de l'extrait de sorgho.

La Demanderesse a réalisé les compositions suivantes :

<u>Composition A (invention):</u>

- Polydiméthyl/méthyl siloxane POE/POP(396/4) (OE/OP 18/18) A 10 % D5 10g
- Cyclopentadiméthylsiloxane 12.5g
- Mélange tocophérols naturels/huile de soja 0.1g
- Propylène glycol 15g
- - Extrait de Sorghum vulgare
  (Sorghum Extract Absorbance >30 de Premier Specialties) 0.7g
- Conservateurs qs
- Eau déminéralisée qsp 100g

<u>Composition B (hors invention)</u>

- Mélange mono/distéarate de glycéryle/alcool cétylstéarylique oxyéthyléné 3g
- Alcool stéarylique 2.5g
- Mélange mono/distéarate de glycéryle/stéarate de polyéthylène glycol 2.5
- Polydiméthylsiloxane 1
- - Extrait de Sorghum vulgare
  (Sorghum Extract Absorbance >30 de Premier Specialties) 0.7g
- Propylène glycol 15g
- Conservateurs qs
- Eau déminéralisée qsp 100g

**<u>Protocole d'évaluation</u> :**

Les compositions A et B ont été appliquées à raison de 2 mg/$cm^2$ sur une zone de 2 * 2 $cm^2$ délimitée sur le dos de l'avant bras dont la couleur de peau caractérisée par l'angle ITA est compris entre 35 et 55.

Les séries de mesures colorimétriques suivantes ont été effectuées à l'aide d'un spectrocolorimètre Minolta CM-508d :

- 1°) avant application de la composition,
- 2°) 30 minutes après l'application,
- 3°) 30 minutes après application et lavage des zones par un coton imbibé de gel douche dilué (gel douche NEUTRALIA à 2%)

Les résultats sont exprimés dans le système (L*, a*, b*) dans lequel L* représente la luminance, a* représente l'axe rouge-vert (-a* = vert, +a* = rouge) et b* représente l'axe jaune-bleu (-b* =bleu, +b* =jaune). Ainsi, a* et b* expriment la nuance de la peau.

Pour l'évaluation de l'intensité de la coloration, on s'intéresse à ΔL* qui traduit l'assombrissement de la couleur : plus ΔL* est négatif, plus la couleur s'est assombrie avec : ΔL* = L* peau non colorée - L* peau colorée

Pour la nuance de la coloration obtenue, on s'intéresse au rapport Δa*/Δb* qui traduit l'équilibre rouge/jaune et donc la nuance avec :

Les résultats obtenus sont rassemblés dans le tableau (I) suivant :

| | Composition A (invention) ΔL* | Composition B (hors invention) ΔL* |
|---|---|---|
| T = 30 minutes | -5 | - 6.2 |
| T = 30 minutes après lavage à l'eau | -3.9 | -1.72 |
| % de perte de ΔL | 22% | 73% |

On constate ainsi que la composition A, qui contient comme agent de coloration l'extrait de sorgho permet d'obtenir une meilleure rémanence au lavage (% de perte du ΔL = 22%) que la composition B qui contient le même agent de coloration à la même concentration (% de perte du ΔL = 73%)

**Comparaison avec une émulsion contenant la DHA :**

La Demanderesse a réalisé la composition suivante :

Composition C (hors invention):

- Polydiméthyl/méthyl siloxane POE/POP(396/4) (OE/OP 18/18) à 10 % D5 10g
- Cyclopentadiméthylsiloxane 12.5g
- Mélange tocophérols naturels/huile de soja 0.1g
- Dihydroxyacétone (DHA) 4g
- Propylène glycol 15
- Conservateurs qs
- Eau déminéralisée qsp 100g

Les compositions A et C ont été appliquées à raison de 2 mg/$cm^2$ sur une zone de 2 * 2 $cm^2$ délimitée sur le dos de l'avant-bras dont la couleur de peau caractérisée par l'angle ITA est compris entre 35 et 55.

Les séries de mesures colorimétriques suivantes ont été effectuées à l'aide d'un colorimètre Minolta CM-508d :

- 1°) avant application de la composition,
- 2°) 30 minutes après l'application,

Les résultats sont exprimés dans le système (L*, a*, b*) dans lequel L* représente la luminance, a* représente l'axe rouge-vert (-a* = vert, +a* = rouge) et b* représente l'axe jaune-bleu (-b* =bleu, +b* =jaune). Ainsi, a* et b* expriment la nuance de la peau.

Pour l'évaluation de l'intensité de la coloration, on s'intéresse à ΔL* qui traduit l'assombrissement de la couleur : plus ΔL* est négatif, plus la couleur s'est assombrie avec :

$$\Delta L^* = L^*\ \text{peau non colorée} - L^*\ \text{peau colorée}$$

Les résultats obtenus sont rassemblés dans le tableau (II) suivant :

Tableau (II) :

| | Composition A (invention) ΔL* | Composition C (hors invention) ΔL* |
|---|---|---|
| T = 30 minutes | -5 | -0.4 |

On constate ainsi que 30 minutes après l'application, la composition C, qui contient à titre d'agent de coloration de la peau, la DHA, n'a conféré qu'une très faible coloration à la peau, puisque la DHA n'a pas encore eu le temps d'agir (ΔL* = - 0,4). En revanche, la composition A selon l'invention a déjà conféré à la peau une coloration significative (ΔL* = - 5).

La composition C contenant la DHA ne permet pas d'obtenir au bout de 30 minutes un assombrissement comparable à celui de la composition A..

## Revendications

1. Emulsion cosmétique ou dermatologique, **caractérisée par le fait qu'**elle comporte :

   (b) au moins une phase aqueuse et
   (b) au moins une phase grasse ;
   (c) au moins un extrait de sorgho ;
   (d) au moins une silicone organomodifiée ; ladite composition ne contenant pas de sel de flavylium non substitué en position 3 et substitué par au moins un radical hydroxyle ou alcoxy.

2. Emulsion selon la revendication 1 où la silicone organomodifiée est choisie parmi :

   (1) les polyorganosiloxanes comportant des groupements oxyalkylénés
   (2) les polyorganosiloxanes comportant des groupements alcoxylés
   (3) les polyorganosiloxanes comportant des groupements anioniques du type 2-hydroxyalkylsulfonate ; 2-hydroxyalkylthiosulfate ;
   (4) les polyorganosiloxanes comportant des groupements thiols ;
   (5) les polyorganosiloxanes comportant des groupements anioniques du type carboxylique
   (6) les polyorganosiloxanes comportant des groupements hydroxylés
   (7) les polyorganosiloxanes comportant des groupements acyloxyalkyle
   (8) les polyorganosiloxanes comportant des groupements aminés substitués ou non.
   (9) les polyorganosiloxanes comportant des groupements hydroxyacylamino,
   (10) les polyorganosiloxanes comportant des groupements de type aryle éventuellement substitué.

3. Emulsion selon la revendication 2, où les polyorganosiloxanes comportant des groupements oxyalkylénés répondent à l'une des formules suivantes :

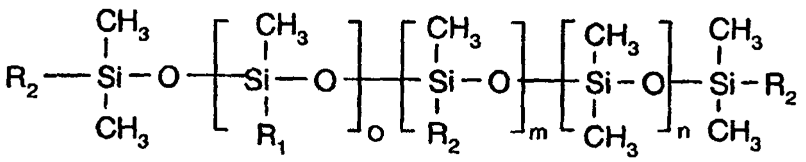
(I)

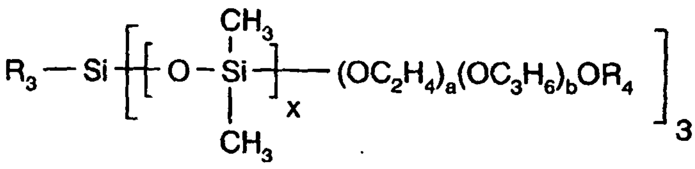
(II)

   - $R_1$, identique ou différent, représente un radical alkyle, linéaire ou ramifié, en $C_1$-$C_{30}$ ou phényle.
   - $R_2$, identique ou différent, $-C_cH_{2c}-(-O-C_2H_4)_a-(-O-C_3H_6)_b-(O-C_4H_8)_d-R_5$
   - $R_3$, $R_4$, identiques ou différents, désignent un radical alkyle, linéaire ou ramifié, en $C_1$-$C_{12}$, et de préférence le radical méthyle,
   - $R_5$, identique ou différent, est choisi parmi un atome d'hydrogène, un radical hydroxyle, un radical alkyle linéaire ou ramifié de 1 à 12 atomes de carbone, un radical alcoxy linéaire ou ramifié de 1 à 6 atomes de carbone, un radical acyloxy linéaire ou ramifié de 2 à 12 atomes de carbone, $NHCH_2CH_2COOM$, aminoalkyle éventuellement substitué sur l'amine, carboxyacyle en $C_1$-$C_{30}$, un groupement phosphono éventuellement substitué,
   - $O-CO-(CH_2)_d-CO_2M$, $-NHCO(CH_2)_dOH$, $-NH_3Y$.
   - M, identique ou différent, désigne un atome d'hydrogène, Na, K, Li, $NH_4$ ou une amine organique,
   - a varie de 0 à 50,
   - b varie de 0 à 50,
   - c varie de 0 à 4,
   - a + b est supérieur ou égal à 1,

- d varie de 0 à 10,
- m varie de 0 à 20,
- n varie de 0 à 500,
- p varie de 0 à 20,
- x varie de 1 à 100,
- Y représente un anion minéral ou organique monovalent.

**4.** Emulsion selon la revendication 2, où les polyorganosiloxanes comportant des groupements anioniques du type carboxylique répondent à la formule (III) suivante :

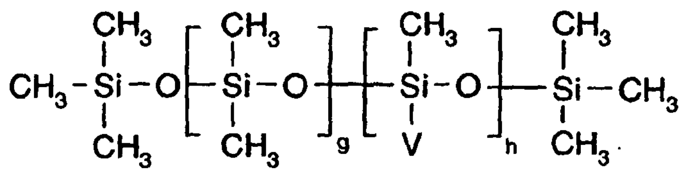

(III)

dans laquelle V est un radical $-(R^1O)_e-R^2-(OR^3)_f-COOM$, dans lequel $R^1$ et $R^3$ désignent indépendamment un radical alkylène linéaire ou ramifié ayant de 2 à 20 atomes de carbone, $R^2$ désigne un radical alkylène linéaire ou ramifié ayant de 1 à 50 atomes de carbone pouvant comprendre un groupement hydroxyle ; e représente 0 ou 1 et f est un nombre allant de 0 à 200 ; M désigne hydrogène, un métal alcalin ou alcalino-terreux, $NH_4$, un groupement ammonium quaternaire te! qu'un groupement mono-, di-, tri- ou tétra (alkyl $C_1$-$C_4$) ammonium ; g et h sont des nombres allant de 0 à 1000, la somme c+d allant de préférence de 2 à 1000.

**5.** Emulsion selon la revendication 2, où les polyorganosiloxanes comportant des groupements hydroxylés répondent à la formule (IV) suivante :

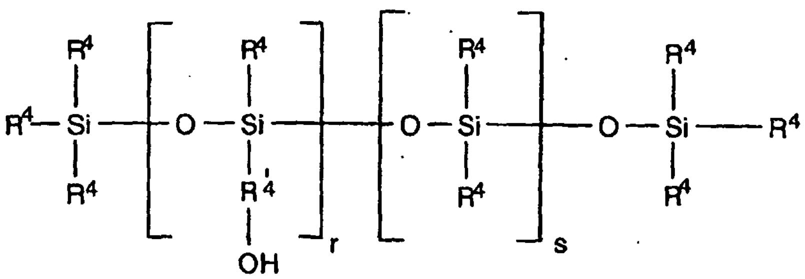

(IV)

dans laquelle les radicaux $R^4$ identiques ou différents sont choisis parmi les radicaux méthyle et phényle ; au moins 60 % en mole des radicaux $R^4$ désignant méthyle ; le radical $R'^4$ est un chaînon alkylène divalent hydrocarboné en $C_2$-$C_{18}$ ; r est compris entre 1 et 30 inclus ; s est compris entre 1 et 150 inclus .

**6.** Emulsion selon la revendication 2, où les polyorganosiloxanes comportant des groupements acyloxyalkyle répondant à la formule (V) :

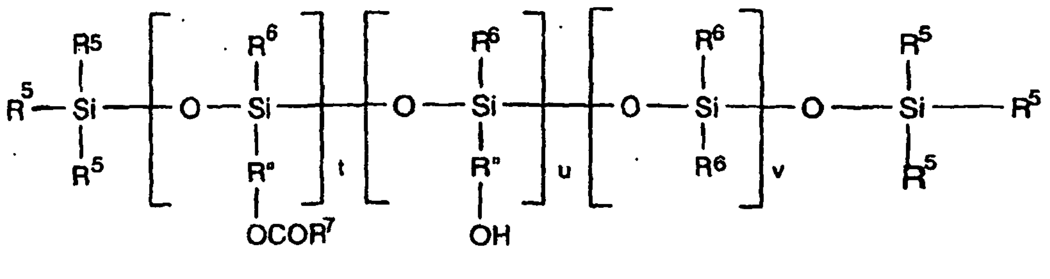

(V)

dans laquelle :

$R^5$ désigne un groupement méthyle, phényle, -$OCOR^7$, hydroxyle, un seul des radicaux $R^5$ par atome de silicium pouvant être OH ;
$R^6$ désigne méthyle, phényle ; au moins 60 % en proportion molaire de l'ensemble des radicaux $R^5$ et $R^6$ désignant méthyle ;
$R^7$ désigne alkyle ou alcényle en $C_8$-$C_{20}$ ;
R" désigne un radical alkylène hydrocarboné divalent, linéaire ou ramifié en $C_2$-$C_{18}$ ;
t est compris entre 1 et 120 inclus ;
u est compris entre 1 et 30 ;
v est égal à 0 ou est inférieur à 0,5 t, t + u étant compris entre 1 et 30 ; les polyorganosiloxanes de formule (V) peuvent contenir des groupements :

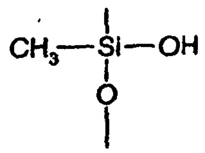

dans des proportions ne dépassant pas 15 % de la somme t + u + v.

**7.** Emulsion selon la revendication 2, où les polyorganosiloxanes comportant des groupements aminés substitués ou non sont choisis parmi :

(a) les polysiloxanes répondant à la formule :

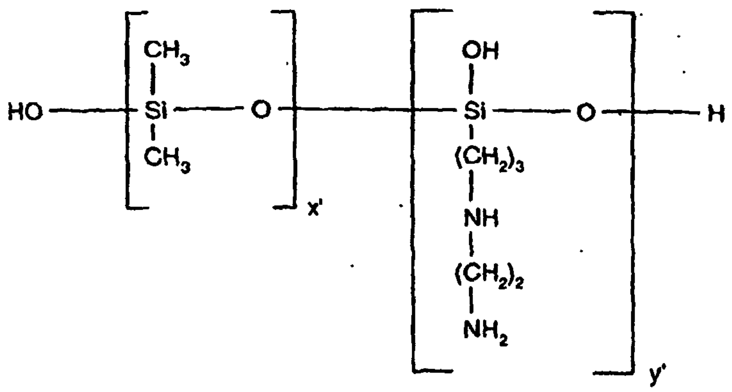

(VI)

dans laquelle x' et y' sont des nombres entiers tels que le poids moléculaire en nombre est compris entre 5 000 et 500 000 ;
(b) les polymères cationiques siliconés répondant à la formule :

$$R^8_iG_{3-i}\text{-}Si(OSiG_2)_k\text{-}(OSiG_jR^8_{2-j})_l\text{-}O\text{-}SiG_{3-i}\text{-}R^8_i \qquad (VII)$$

dans laquelle :

G est un atome d'hydrogène, ou un groupement phényle, OH, ou alkyle en $C_1$-$C_8$, par exemple méthyle,
i désigne le nombre 0 ou un nombre entier de 1 à 3, en particulier 0,
j désigne 0 ou 1, et en particulier 1,
k et l sont des nombres tels que la somme (k+l) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
$R^8$ est un radical monovalent de formule -$C_qH_{2q}L$ dans laquelle q est un nombre de 2 à 8 et L est un groupement aminé éventuellement quaternisé choisi parmi les groupements :

$$-NR^9\text{-}CH_2\text{-}CH_2\text{-}N(R^9)_2$$

$-N(R^9)_2$

$-N^{\oplus}(R^9)_3A^-$

$-N(R^9)-CH_2-CH_2-N^{\oplus}R^9H_2A^-$,

dans lesquels $R^9$ désigne hydrogène, phényle, benzyle, ou un radical hydrocarboné saturé monovalent, par exemple un radical alkyle ayant de 1 à 20 atomes de carbone et $A^-$ représente un ion halogénure ;
(c) les polymères cationiques siliconés répondant à la formule :

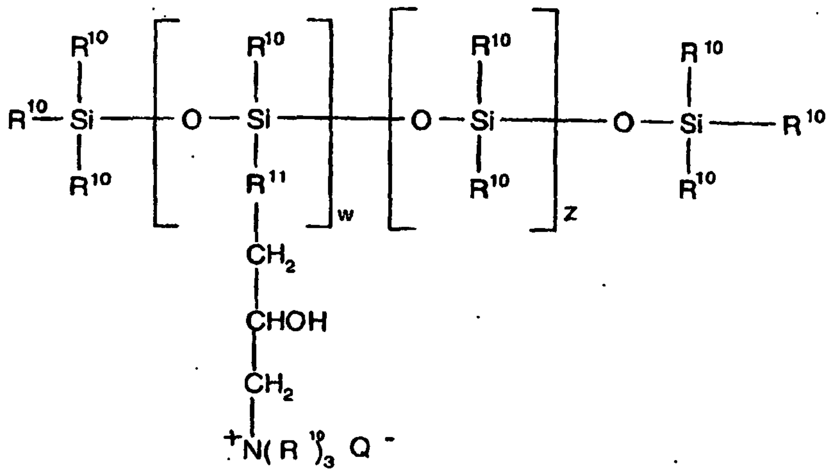

(VIII)

dans laquelle :

$R^{10}$ représente un radical hydrocarboné monovalent ayant de 1 à 18 atomes de carbone, et en particulier un radical alkyle en $C_1$-$C_{18}$, ou alcényle en $C_2$-$C_{18}$, par exemple méthyle ;
$R^{11}$ représente un radical hydrocarboné divalent, et en particulier un radical alkylène en $C_1$-$C_{18}$ ou un radical alkylèneoxy divalent en $C_1$-$C_{18}$, par exemple en $C_1$-$C_8$ ;
$Q^-$ est un ion halogénure ;
w représente une valeur statistique moyenne de 2 à 20 et en particulier de 2 à 8 ;
z représente une valeur statistique moyenne de 20 à 200 et en particulier de 20 à 50.

**8.** Emulsion selon l'une quelconque des revendications 1 à 7, où les silicones organomodifiées sont présentes dans des concentrations allant de 0,1 à 40 % par rapport au poids total de la composition, et plus préférentiellement en une quantité allant de 0,5 à 20 %.

**9.** Emulsion selon l'une quelconque des revendications 1 à 8, **caractérisée par le fait qu'**elle se présente sous forme d'émulsion eau-dans-silicone.

**10.** Emulsion selon la revendication 9, où la silicone organomodifiée est choisi parmi celles comportant des groupements oxyalkylénés telles que définies dans la revendication 2 ou 3.

**11.** Emulsion selon l'une quelconque des revendications 1 à 10, selon laquelle ledit extrait de sorgho est présent dans une quantité efficace permettant d'obtenir au bout de 30 minutes après application sur une peau claire à raison de 2mg/cm2 un assombrissement **caractérisé** dans le système de mesure colorimétrique L* a* b* par un ΔL* allant de - 0,5 à -20.

**12.** Emulsion selon la revendication 11 où le ΔL* varie de - 0,5 à -15.

**13.** Emulsion selon l'une quelconque des revendications 1 à 12, selon laquelle ledit extrait de sorgho est présent dans une quantité efficace permettant d'obtenir au bout de 30 minutes après application sur une peau claire à raison de

2mg/cm$^2$ une coloration définie dans le système colorimétrique L* a* b* par un rapport $\Delta$a*/$\Delta$b* allant de 0,5 à 3.

**14.** Emulsion selon la revendication 13, où le rapport $\Delta$a*/$\Delta$b* varie de 0,8 à 2.

**15.** Emulsion selon l'une quelconque des revendications 1 à 14 où ledit extrait de sorgho est obtenu à partir de la plante entière, de tiges, de graines ou de feuilles du genre Sorghum, à l'état frais ou sec.

**16.** Emulsion selon la revendication 15 où les espèces du Sorghum sont choisies parmi le Sorghum bicolor, de Sorghum caudatum, de Sorghum nervosum, de Sorghum durra, de Sorghum vulgare ou les Sorghum en association avec du Colletotrichum Graminicola.

**17.** Emulsion selon l'une quelconque des revendications 1 à 16 où ledit extrait de sorgho est obtenu à partir de la plante entière, de tiges, de graines ou de feuilles de Sorghum vulgare.

**18.** Composition selon l'une quelconque des revendications 1 à 17 où l'extrait de sorgho est susceptible d'être obtenu selon un procédé comprenant les étapes suivantes :

(a) une extraction des parties à partir de la plante entière, de tiges, de graines ou de feuilles de Sorghum dans un milieu aqueux pouvant contenir en plus au moins un solvant organique ;
(b) une macération dans un milieu alcalin présentant un pH de l'ordre 11-12.
(c) éventuellement une précipitation du milieu de macération par addition d'un acide de telle sorte à atteindre un pH de l'ordre de 1-2.

**19.** Emulsion selon l'une quelconque des revendications 1 à 18 où la concentration en extrait de Sorgho conforme à l'invention varie 0,0001 à 10%, et préférentiellement de 0,001 à 5% en poids, par rapport au poids total de la composition.

**20.** Composition selon l'une quelconque des revendications 1 à 19, contenant en plus au moins un autobronzant mono ou polycarbonylé.

**21.** Composition selon la revendication 20, où le ou les autobronzants mono ou polycarbonylés sont choisis parmi l'isatine, l'alloxane, la ninhydrine, le glycéraldéhyde, l'aldéhyde mésotartrique, la glutaraldéhyde, l'érthrulose, les dérivés de pyrazoline-4,5-diones, la dihydroxyacétone (DHA), les dérivés de 4,4-dihydroxypyrazolin-5-ones ; ces autobronzants pouvant être associés ou non à des colorants directs ou des dérivés indoliques.

**22.** Composition selon la revendication 21, où l'autobronzant est la dihydroxyacétone (DHA).

**23.** Composition selon l'une quelconque des 20 à 22, où l'autobronzant est présent dans des proportions allant de 0,1 à 10% en poids par rapport au poids total de la composition.

**24.** Composition selon l'une quelconque des revendications 1 à 23, contenant en plus au moins un agent filtrant les radiations UV.

**25.** Composition selon la revendication 24, où ledit agent filtrant les radiations UV est choisi parmi les filtres UV organiques ou les agents filtrant les radiations UV minéraux.

**26.** Composition selon la revendication 25, où le ou les filtres UV organiques sont hydrosolubles, liposolubles ou insolubles dans les solvants usuels cosmétiques.

**27.** Composition selon la revendication 26, où le ou les filtres UV organiques sont choisis parmi les anthranilates ; les dérivés cinnamiques ; les dérivés de dibenzoylméthane ; les dérivés salicyliques, les dérivés du camphre ; les dérivés de triazine ; les dérivés de la benzophénone ; les dérivés de $\beta,\beta'$-diphénylacrylate, les dérivés de benzotriazole, les dérivés de benzimidazole ; les imadazolines ; les dérivés bis-benzoazolyle ; les dérivés de l'acide p-aminobenzoïque (PABA) ; les dérivés de méthylène bis-(hydroxyphényl benzotriazole) ; les polymères filtres et silicones filtres ; les dimères dérivés d'$\alpha$-alkylstyrène ; les dérivés de 4,4-diarylbutadiène et leurs mélanges.

**28.** Composition selon la revendication 27, où le ou les agents filtrants les radiations UV organiques sont choisis parmi :

- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalylidene Dicamphor Sulfonic,
- Benzophenone-3,
- Benzophenone-4,
- Benzophenone-5,
- 4-Methylbenzylidene camphor,
- Benzimidazilate,
- Anisotriazine,
- Ethylhexyl triazone,
- Diethylhexyl Butamido Triazone,
- la 2,4,6-tris-(4'-amino benzalmalonate de diisobutyle)-s- triazine.
- Methylene bis-Benzotriazolyl Tetramethylbutylphenol,
- Drometrizole Trisiloxane,
- 1,1-dicarboxy (2,2'-diméthyl-propyl)-4,4-diphénylbutadiène

et leurs mélanges.

**29.** Composition selon la revendication 25, où le ou les agents filtrant les radiations UV minéraux sont choisis parmi les pigments ou les nanopigments d'oxydes métalliques enrobés ou non.

**30.** Composition selon la revendication 29, où le ou les agents filtrant les radiations UV minéraux sont choisis parmi les nanopigments d'oxyde de titane, de fer, de zinc, de zirconium ou de cérium, enrobés ou non.

**31.** Composition selon l'une quelconque des revendications 24 à 30, où le ou les agents filtrant les radiations UV sont présents dans des proportions allant de 0,1 à 15% en poids par rapport au poids total de la composition.

**32.** Procédé de coloration artificielle proche du bronzage naturel de la peau, **caractérisé en ce qu'**il consiste à appliquer sur celle-ci une quantité efficace d'une émulsion telle que définie à l'une quelconque des revendications 1 à 31.

**33.** Utilisation d'au moins un extrait de sorgho et d'au moins une silicone organomodifiée tels que définis dans les revendications précédentes dans une émulsion cosmétique ne contenant pas de sel de flavylium non substitué en position 3 et substitué par au moins un radical hydroxyle ou alcoxy, dans le but de conférer à la peau une coloration artificielle proche du bronzage naturel.

## Patentansprüche

**1.** Kosmetische oder dermatologische Emulsion, **dadurch gekennzeichnet, dass** sie enthält:

(a) mindestens eine wässrige Phase und
(b) mindestens eine Fettphase;
(c) mindestens einen Sorghum-Extrakt; und
(d) mindestens ein organomodifiziertes Silicon;

wobei die Zusammensetzung kein in 3-Stellung unsubstituiertes und mit mindestens einer Hydroxy- oder Alkoxygruppe substituiertes Flavyliumsalz enthält.

**2.** Emulsion nach Anspruch 1, wobei das organomodifizierte Silicon ausgewählt ist unter:

(1) Polyorganosiloxanen mit alkoxylierten Gruppen,
(2) Polyorganosiloxanen, die Alkoxygruppen enthalten,
(3) Polyorganosiloxanen, die anionische Gruppen vom Typ 2-Hydroxyalkylsulfonat; 2-Hydroxyalkylthiosulfat enthalten,
(4) Polyorganosiloxanen, die Thiolgruppen enthalten,

(5) Polyorganosiloxanen, die anionische Gruppen vom Carboxytyp enthalten,
(6) Polyorganosiloxanen, die hydroxylierte Gruppen enthalten,
(7) Polyorganosiloxanen, die Acyloxyalkylgruppen enthalten,
(8) Polyorganosiloxanen, die substituierte oder unsubstituierte aminierte Gruppen enthalten,
(9) Polyorganosiloxanen, die Hydroxyacylaminogruppen enthalten,
(10) Polyorganosiloxanen, die Gruppen vom Aryltyp enthalten, die gegebenenfalls substituiert sind.

**3.** Emulsion nach Anspruch 2, wobei die Polyorganosiloxane, die alkoxylierte Gruppen enthalten, einer der folgenden Formeln entsprechen:

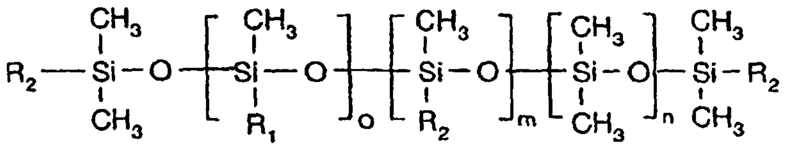

(I)

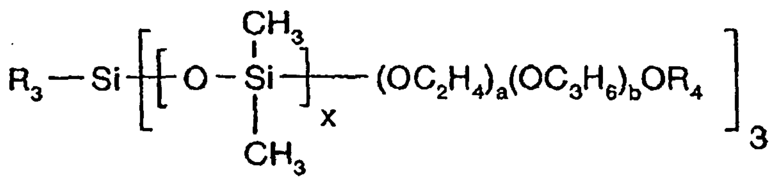

(II),

worin bedeuten:

- die Gruppen $R_1$, die gleich oder verschieden sind, eine geradkettige oder verzweigte $C_{1-30}$-Alkylgruppe oder Phenyl,
- die Gruppen $R_2$, die gleich o der verschieden sind,

$$-C_cH_{2c}-(-O-C_2H_4)_a-(-O-C_3H_6)_b-(O-C_4H_8)_d-R_5,$$

- die Gruppen $R_3$ und $R_4$, die gleich oder verschieden sind, eine geradkettige oder verzweigte $C_{1-12}$-Alkylgruppe und vorzugsweise die Methylgruppe,
- die Gruppen $R_5$, die gleich oder verschieden sind, ein Wasserstoffatom, eine Hydroxygruppe, eine geradkettige oder verzweigte Alkylgruppe mit 1 bis 12 Kohlenstoffatomen, eine geradkettige oder verzweigte Alkoxygruppe mit 1 bis 6 Kohlenstoffatomen, eine geradkettige oder verzweigte Acyloxygruppe mit 2 bis 12 Kohlenstoffatomen, $NHCH_2CH_2COOM$, eine Aminoalkylgruppe, deren Aminogruppe gegebenenfalls substituiert ist, eine $C_{1-30}$-Carboxyacylgruppe, eine gegebenenfalls substituierte Phosphonogruppe,

$$-O-CO-(CH_2)_d-CO_2M,\ -NHCO(CH_2)_dOH,\ -NH_3Y,$$

- die Gruppen M, die gleich oder verschieden sind, ein Wasserstoffatom, Na, K, Li, $NH_4$ oder ein organisches Amin,
- a liegt im Bereich von 0 bis 50,
- b liegt im Bereich von 0 bis 50,
- c liegt im Bereich von 0 bis 4,
- a + b bedeutet 1 oder liegt über 1,
- d liegt im Bereich von 0 bis 10,
- m liegt im Bereich von 0 bis 20,
- n liegt im Bereich von 0 bis 500,
- p liegt im Bereich von 0 bis 20,
- x liegt im Bereich von 1 bis 100,
- Y bedeutet ein einwertiges anorganisches oder organisches Anion.

**4.** Emulsion nach Anspruch 2, wobei die Polyorganosiloxane, die anionische Gruppen vom Carboxytyp enthalten, der folgenden Formel (III) entsprechen:

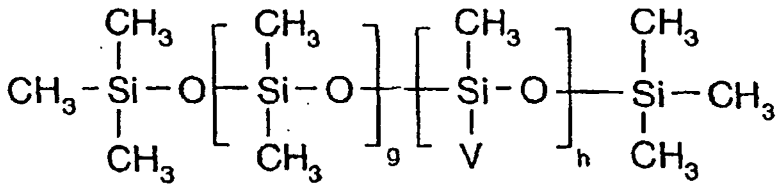

(III),

worin V eine Gruppe $-(R^1O)_e-R^2-(OR^3)_f-COOM$ bedeutet, wobei $R^1$ und $R^3$ unabhängig voneinander eine geradkettige oder verzweigte Alkylengruppe mit 2 bis 20 Kohlenstoffatomen bedeuten, $R^2$ eine geradkettige oder verzweigte Alkylengruppe mit 1 bis 50 Kohlenstoffatomen ist, die eine Hydroxygruppe enthalten kann; e 0 oder 1 bedeutet und f eine Zahl im Bereich von 0 bis 200 ist; M Wasserstoff, ein Alkalimetall, ein Erdalkalimetall, $NH_4$ oder eine quartäre Ammoniumgruppe bedeutet, beispielsweise eine Mono-, Di-, Tri- oder Tetraalkyl($C_1$-$_4$)ammoniumgruppe; g und h Zahlen von 0 bis 1 000 bedeuten, wobei die Summe c+d vorzugsweise im Bereich von 2 bis 1 000 liegt.

**5.** Emulsion nach Anspruch 2, wobei die Polyorganosiloxane, die hydroxylierte Gruppen enthalten, der folgenden Formel (IV) entsprechen:

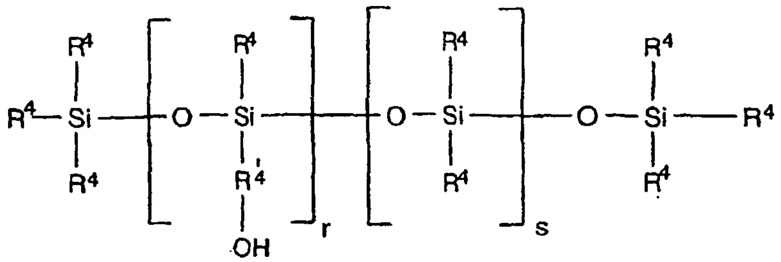

(IV),

worin die Gruppen $R^4$, die gleich oder verschieden sind, unter den Gruppen Methyl und Phenyl ausgewählt sind, wobei mindestens 60 Mol-% der Gruppen $R^4$ Methyl bedeuten; die Gruppe $R'^4$ ist eine zweiwertige Alkylenkette mit 2 bis 18 Kohlenstoffatomen; r liegt im Bereich von 1 bis 30, wobei die Grenzen eingeschlossen sind, s liegt im Bereich von 1 bis 150, wobei die Grenzen eingeschlossen sind.

**6.** Emulsion nach Anspruch 2, wobei die Polyorganosiloxane, die Acyloxyalkylgruppen enthalten, der Formel (V) entsprechen:

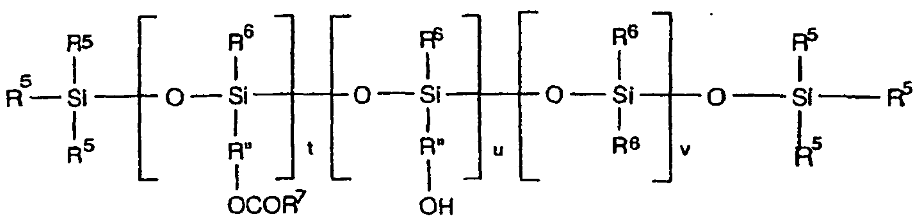

(V),

worin bedeuten:

R5 Methyl, Phenyl, $-OCOR^7$, Hydroxy, wobei eine Gruppe $R^5$ pro Siliciumatom OH bedeuten kann;
R6 Methyl, Phenyl, wobei mindestens 60 Mol-% der gesamten Gruppen $R^5$ und $R^6$ Methyl bedeuten:

$R^7$ Alkyl oder Alkenyl mit 8 bis 20 Kohlenstoffatomen;

R" eine geradkettige oder verzweigte, zweiwertige Alkylengruppe auf Kohlenwasserstoffbasis mit 2 bis 18 Kohlenstoffatomen;
t liegt im Bereich von 1 bis 120, wobei die Grenzen eingeschlossen sind;
u liegt im Bereich von 1 bis 30;
v bedeutet 0 oder liegt unter 0,5 t, wobei t + u im Bereich von 1 bis 30 liegt; die Polyorganosiloxane der Formel (V) können auch Gruppen:

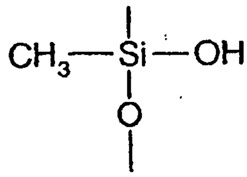

in Anteilen enthalten, die 15 % der Summe t + u + v nicht übersteigen.

**7.** Emulsion nach Anspruch 2, wobei die Polyorganosiloxane, die substituierte oder unsubstituierte aminierte Gruppen enthalten, ausgewählt sind unter:

(a) Polysiloxanen der folgenden Formel:

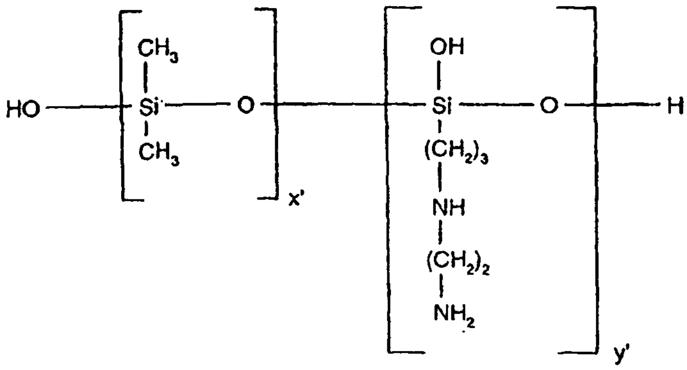
(VI),

worin x' und y' ganze Zahlen bedeuten, die so ausgewählt sind, dass die zahlenmittlere Molmasse im Bereich von 5 000 bis 500 000 liegt;
(b) kationischen siliconierten Polymeren der folgenden Formel

$R^8_iG_{3-i}$-Si$(OSiG_2)_k$-$(OSiG_jR^8_{2-j})_l$-O-Si$G_{3-i}$-$R^8_i$ (VII),

worin bedeuten:

G ein Wasserstoffatom, eine Phenylgruppe, OH oder $C_{1-8}$-Alkyl, beispielsweise Methyl,
i 0 oder eine ganze Zahl von 1 bis 3 und insbesondere 0,
j 0 oder 1 und insbesondere 1,
k und l Zahlen, die so gewählt sind, dass die Summe (k+l) insbesondere im Bereich von 1 bis 2 000 und besonders 50 bis 150 liegen kann, wobei n eine Zahl von 0 bis 1 999 und
insbesondere 49 bis 149 bedeuten und m eine ganze Zahl von 1 bis 2 000 und insbesondere 1 bis 10 bedeuten kann;:
$R^8$ eine einwertige Gruppe der Formel $-C_qH_{2q}L$, wobei q eine Zahl von 2 bis 8 ist und L eine gegebenenfalls quaternisierte aminierte Gruppe ist, die unter den folgenden Gruppen ausgewählt ist:

$-NR^9-CH_2-CH_2-N(R^9)_2$

$-N(R^9)_2$

$-N^{\oplus}(R^9)_3A^-$

$-N((R^9)-CH_2-CH_2-N^{\oplus}R^9H_2A^-$

wobei $R^9$ Wasserstoff, Phenyl, Benzyl oder eine gesättigte einwertige Kohlenwasserstoffgruppe bedeutet, beispielsweise eine Alkylgruppe mit 1 bis 20 Kohlenstoffatomen, und $A^-$ ein Halogenid ist;
(c) kationischen siliconierten Polymeren der folgenden Formel:

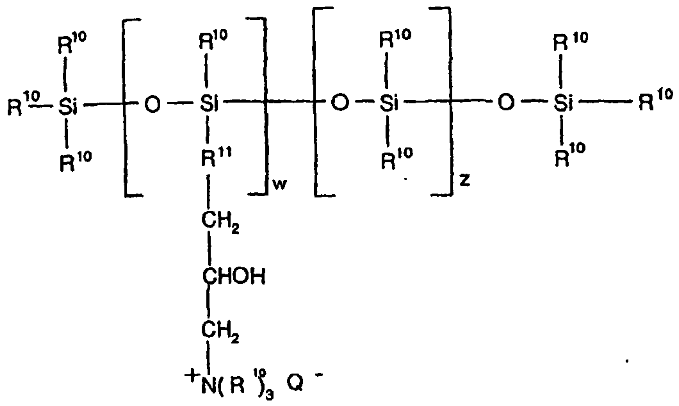

(VIII),

worin bedeuten:

$R^{10}$ bedeutet eine einwertige Kohlenwasserstoffgruppe mit 1 bis 18 Kohlenstoffatomen, insbesondere $C_{1-18}$-Alkyl oder $C_{2-18}$-Alkenyl, beispielsweise Methyl;
$R^{11}$ bedeutet eine zweiwertige Kohlenwasserstoffgruppe, insbesondere eine $C_{1-18}$-Alkylengruppe oder eine zweiwertige $C_{1-18}$-Alkylenoxygruppe, beispielsweise eine Gruppe mit 1 bis 8 Kohlenstoffatomen;
$Q^-$ ist ein Halogenid, insbesondere Chlorid,
w bedeutet einen statistischen Mittelwert von 2 bis 20 und insbesondere 2 bis 8;
z ist ein statistischer Mittelwert von 20 bis 200 und insbesondere 20 bis 50.

**8.** Emulsion nach einem der Ansprüche 1 bis 7, wobei die organomodifizierten Silicone in Konzentrationen von 0,1 bis 40 %, bezogen auf das Gesamtgewicht der Zusammensetzung, und noch bevorzugter in einer Menge von 0,5 bis 20 % enthalten sind.

**9.** Emulsion nach einem der Ansprüche 1 bis 8, **dadurch gekennzeichnet, dass** sie in Form einer Wasser-in-Silicon-Emulsion vorliegt.

**10.** Emulsion nach Anspruch 9, wobei das organomodifizierte Silicon unter den Verbindungen ausgewählt ist, die alkoxylierte Gruppen enthalten, wie sie in den Ansprüche 2 oder 3 definiert wurden.

**11.** Emulsion nach einem der Ansprüche 1 bis 10, wobei der Sorghum-Extrakt in einer Menge vorliegt, die wirksam ist, um 30 Minuten nach dem Aufbringen auf einen hellen Hauttyp in einer Menge von 2 $mg/cm^2$ ein Dunklerwerden zu erzielen, das im kolorimetrischen System L*a*b* durch einen $\Delta L^*$-Wert von -0,5 bis -20 **gekennzeichnet** ist.

**12.** Emulsion nach Anspruch 11, wobei $\Delta L^*$ im Bereich von -0,5 bis -15 liegt.

**13.** Emulsion nach einem der Ansprüche 1 bis 12, wobei der Sorghum-Extrakt in einer Menge enthalten ist, die wirksam ist, um 30 min nach dem Auftragen auf einen hellen Hauttyp in einer Menge von 2 $mg/cm^2$ eine im kolorimetrischen L*a*b*-System durch ein Verhältnis $\Delta a^*/\Delta b^*$ definierte Färbung von 0,5 bis 3 zu erzielen.

**14.** Emulsion nach Anspruch 13, wobei das Verhältnis $\Delta a^*/\Lambda b^*$ im Bereich von 0,8 bis 2 liegt.

**15.** Emulsion nach einem der Ansprüche 1 bis 14, wobei der Sorghum-Extrakt aus der ganzen Pflanze, den Stängeln, Samen oder Blättern von Pflanzen der Gattung Sorghum im frischen oder trockenen Zustand erhalten wird.

**16.** Emulsion nach Anspruch 15, wobei die Sorghum-Arten unter *Sorghum bicolor, Sorghum caudatum, Sorghum nervosum, Sorghum durra, Sorghum vulgare* oder Sorghum in Kombination mit Colletotrichum Graminicola ausgewählt sind.

**17.** Emulsion nach einem der Ansprüche 1 bis 16, wobei der Sorghum-Extrakt aus der ganzen Pflanze, den Stängeln, Samen oder Blättern von Sorghum vulgare gewonnen wird.

**18.** Zusammensetzung nach einem der Ansprüche 1 bis 17, wobei der Sorghum-Extrakt nach einem Verfahren erhältlich ist, das die folgenden Schritte umfasst:

(a) Extraktion von Teilen aus der gesamten Pflanze, den Stängeln, den Samen oder den Blättern von Sorghum in einem wässrigen Medium, das ferner mindestens ein organisches Lösungsmittel enthalten kann;
(b) Mazeration in einem alkalischen Medium, das einen pH-Wert in der Größenordnung von 11 bis 12 aufweist;
(c) gegebenenfalls Fällen des Mazerationsmediums durch Zusatz einer Säure, die so zugegeben wird, dass der pH-Wert in der Größenordnung von 1 bis 2 eingestellt wird.

**19.** Emulsion nach einem der Ansprüche 1 bis 18, wobei die Konzentration des erfindungsgemäßen Sorghum-Extrakts im Bereich von 0,0001 bis 10 % und vorzugsweise 0,001 bis 5 Gew.-%, bezogen auf das Gesamtgewicht der Zusammensetzung, liegt.

**20.** Zusammensetzung nach einem der Ansprüche 1 bis 19, die ferner mindestens ein mono- oder polycarboxyhaltiges Selbstbräunungsmittel enthält.

**21.** Zusammensetzung nach Anspruch 20, wobei das mono- oder polycarboxyhaltige Selbstbräunungsmittel oder die mono- oder carboxyhaltigen Selbstbräunungsmittel unter Isatin, Alloxan, Ninhydrin, Glycerinaldehyd, Mesoweinsäurealdehyd, Glutaraldehyd, Erythrulose, Pyrazolin-4,5-dionderivaten, Dihydroxyaceton (DHA), 4,4-Dihydroxypyrazolin-5-onderivaten ausgewählt sind, wobei diese Selbstbräunungsmittel gegebenenfalls mit Direktfarbstoffen oder Indolderivaten kombiniert werden können.

**22.** Zusammensetzung nach Anspruch 21, wobei das Selbstbräunungsmittel das Dihydroxyaceton (DHA) ist.

**23.** Zusammensetzung nach einem der Ansprüche 20 bis 22, wobei das Selbstbräunungsmittel in Anteilen von 0,1 bis 10 Gew.-%, bezogen auf das Gesamtgewicht der Zusammensetzung, vorliegt.

**24.** Zusammensetzung nach einem der Ansprüche 1 bis 23, die ferner mindestens ein UV-Filter enthält.

**25.** Zusammensetzung nach Anspruch 24, wobei das UV-Filter unter den organischen UV-Filtern oder anorganischen Stoffen, die UV-Strahlung filtern, ausgewählt ist.

**26.** Zusammensetzung nach Anspruch 25, wobei das oder die organischen UV-Filter wasserlöslich, fettlöslich oder in üblichen kosmetischen Lösungsmitteln unlöslich sind.

**27.** Zusammensetzung nach Anspruch 26, wobei das oder die organischen UV-Filter unter Anthranilaten; Zimtsäurederivaten; Dibenzoylmethanderivaten; Salicylsäurederivaten; Campherderivaten; Triazinderivaten; Benzophenonderivaten; β,β'-Diphenylacrylatderivaten, Benzotriazolderivaten, Benzimidazolderivaten; Imidazolinen; Bis-benzoazolylderivaten; p-Aminobenzoesäurederivaten (PABA); Methylen-bis(hydroxyphenylbenzotriazol)-derivaten; Polymerfiltern und Siliconfiltern; von α-Alkylstyrol abgeleiteten Dimeren; 4,4-Diarylbutadienderivaten und deren Gemischen ausgewählt sind.

**28.** Zusammensetzung nach Anspruch 27, wobei das oder die organischen UV-Filter ausgewählt sind unter:

- Ethylhexyl Salicylate,
- Butyl Methoxydibenzoylmethane,
- Ethylhexyl Methoxycinnamate,
- Octocrylene,

- Phenylbenzimidazole Sulfonic Acid,
- Terephthalylidene Dicamphor Sulfonic,
- Benzophenone-3,
- Benzophenone-4,
- Benzophenone-5,
- 4-Methylbenzylidene camphor,
- Benzimidazilate,
- Anisotriazine,
- Ethylhexyl Triazone,
- Diethylhexyl Butamino Triazone,
- 2,4,6-Tris-(düsobutyl-4'-amino-benzalmalonat)-2-triazin,
- Methylene-bis-benzotriazolyl-tetramethylbutylphenol,
- Drometrizole Trisiloxane,
- 1,1-Dicarboxy-(2,2'-dimethyl-propyl)-4,4-diphenylbutadien,

und deren Gemischen.

**29.** Zusammensetzung nach Anspruch 25, wobei das oder die anorganischen UV-Filter unter den Pigmenten oder Nanopigmenten von Metalloxiden, die umhüllt oder nicht umhüllt sind, ausgewählt sind.

**30.** Zusammensetzung nach Anspruch 29, wobei das oder die anorganischen UV-Filter unter den Nanopigmenten von Titanoxid, Eisenoxid, Zinkoxid, Zirconiumoxid oder Ceroxid, die umhüllt oder nicht umhüllt sind, ausgewählt sind.

**31.** Zusammensetzung nach einem der Ansprüche 24 bis 30, wobei das oder die UV-Filter in Mengenanteilen von 0,1 bis 15Gew.-%, bezogen auf das Gesamtgewicht der Zusammensetzung, enthalten sind.

**32.** Verfahren zur künstlichen Färbung der Haut, die der natürlichen Bräunung nahe kommt, **dadurch gekennzeichnet, dass** es darin besteht, auf die Haut eine wirksame Menge einer Emulsion nach einem der Ansprüche 1 bis 31 aufzutragen.

**33.** Verwendung mindestens eines Sorghum-Extrakts und mindestens eines organomodifizierten Silicons nach einem der vorhergehenden Ansprüche in einer kosmetischen Emulsion, die kein in 3-Stellung unsubstituiertes und mit mindestens einer Hydroxy- oder Alkoxygruppe substituiertes Flavyliumsalz enthält, um der Haut eine künstliche Färbung zu geben, die der natürlichen Bräunung nahe kommt.

## Claims

**1.** Cosmetic or dermatological emulsion, **characterized in that** it comprises:

(a) at least one aqueous phase and
(b) at least one fatty phase;
(c) at least one sorghum extract;
(d) at least one organomodified silicone; the said composition not containing a flavylium salt which is unsubstituted in position 3 and which is substituted with at least one hydroxyl or alkoxy radical.

**2.** Emulsion according to Claim 1, in which the organomodified silicone is chosen from:

(1) polyorganosiloxanes comprising oxyalkylenated groups;
(2) polyorganosiloxanes comprising alkoxylated groups;
(3) polyorganosiloxanes comprising anionic groups such as 2-hydroxyalkylsulphonate; 2-hydroxyalkylthiosulphate;
(4) polyorganosiloxanes comprising thiol groups;
(5) polyorganosiloxanes comprising anionic groups of carboxylic type;
(6) polyorganosiloxanes comprising hydroxylated groups;
(7) polyorganosiloxanes comprising acyloxyalkyl groups;
(8) polyorganosiloxanes comprising substituted or unsubstituted amine groups;
(9) polyorganosiloxanes comprising hydroxyacylamino groups;

(10) polyorganosiloxanes comprising optionally substituted aryl groups.

3. Emulsion according to Claim 2, in which the polyorganosiloxanes comprising oxyalkylenated groups correspond to one of the following formulae:

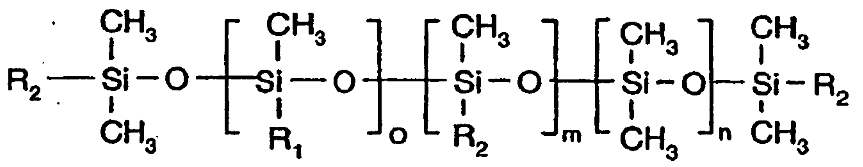

(I)

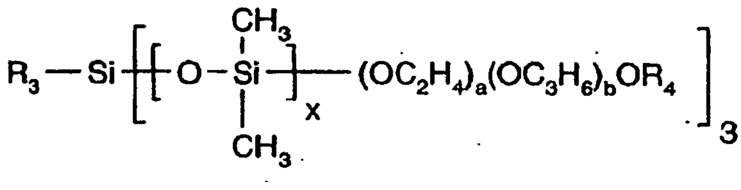

(II)

- $R_1$, which may be identical or different, represents a linear or branched $C_1$-$C_{30}$ alkyl radical or phenyl radical,
- $R_2$, which may be identical or different, represents

$$-C_cH_{2c}-(-O-C_2H_4)_a-(-O-C_3H_6)_b-(O-C_4H_8)_d-R_5,$$

- $R_3$ and $R_4$, which may be identical or different, denote a linear or branched $C_1$-$C_{12}$ alkyl radical and preferably a methyl radical,
- $R_5$, which may be identical or different, is chosen from a hydrogen atom, a hydroxyl radical, a linear or branched alkyl radical of 1 to 12 carbon atoms, a linear or branched alkoxy radical of 1 to 6 carbon atoms, a linear or branched acyloxy radical of 2 to 12 carbon atoms, $NHCH_2CH_2COOM$, aminoalkyl optionally substituted on the amine, carboxy($C_1$-$C_{30}$)acyl, an optionally substituted phosphono group, $-O-CO-(CH_2)_d-CO_2M$, $-NHCO(CH_2)_dOH$ or $-NH_3Y$,
- M, which may be identical or different, denotes a hydrogen atom, Na, K, Li, $NH_4$ or an organic amine,
- a ranges from 0 to 50,
- b ranges from 0 to 50,
- c ranges from 0 to 4,
- a + b is greater than or equal to 1,
- d ranges from 0 to 10,
- m ranges form 0 to 20,
- n ranges from 0 to 500,
- p ranges from 0 to 20,
- x ranges from 1 to 100.
- Y represents an inorganic or organic monovalent anion.

4. Emulsion according to Claim 2, in which the polyorganosiloxanes comprising anionic groups of the carboxylic type correspond to formula (III) below:

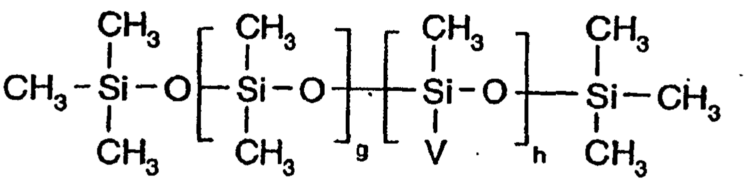

(III)

in which V is a radical $-(R^1O)_e-R^2-(OR^3)_f-COOM$, in which $R^1$ and $R^3$ independently denote a linear or branched alkylene radical containing from 2 to 20 carbon atoms, $R^2$ denotes a linear or branched alkylene radical containing

from 1 to 50 carbon atoms which may comprise a hydroxyl group; e represents 0 or 1 and f is a number ranging from 0 to 200; M denotes hydrogen, an alkali metal or alkaline-earth metal, $NH_4$ or a quaternary ammonium group such as a mono-, di-, tri- or tetra($C_1$-$C_4$alkyl)ammonium group; g and h are numbers ranging from 0 to 1000. the sum c+d preferably ranging from 2 to 1000.

**5.** Emulsion according to Claim 2, in which the polyorganosiloxanes comprising hydroxylated groups correspond to formula (IV) below:

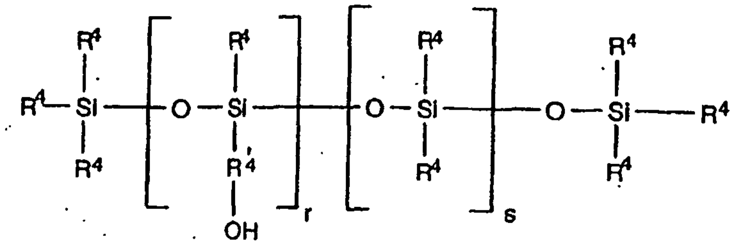 (IV)

in which the radicals $R^4$, which may be identical or different, are chosen from methyl and phenyl radicals, at least 60 mol% of the radicals $R^4$ denoting methyl; the radical $R'^4$ is a divalent hydrocarbon-based $C_2$-$C_{18}$ alkylene chain unit; r is between 1 and 30 inclusive; s is between 1 and 150 inclusive.

**6.** Emulsion according to Claim 2, in which the polyorganosiloxanes comprising acyloxyalkyl groups correspond to formula (V):

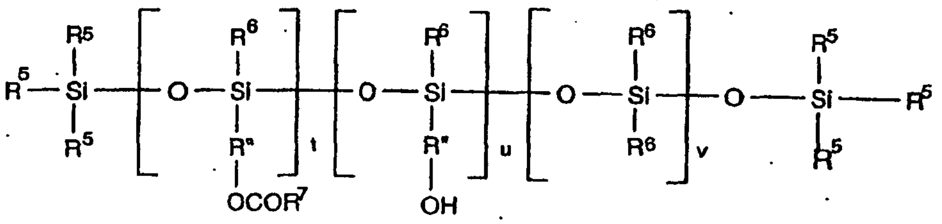 (V)

in which:

$R^5$ denotes a methyl, phenyl, $-OCOR^7$ or hydroxyl group, only one of the radicals $R^5$ per silicon atom possibly being OH;
$R^6$ denotes methyl or phenyl, at least 60 mol% of all of the radicals $R^5$ and $R^6$ denoting methyl;
$R^7$ denotes $C_8$-$C_{20}$ alkyl or alkenyl;
R" denotes a linear or branched divalent hydrocarbon-based $C_2$-$C_{18}$ alkylene radical;
t is between 1 and 120 inclusive;
u is between 1 and 30;
v is equal to 0 or is less than 0.5 t, t + u being between 1 and 30; the polyorganosiloxanes of formula (V) may contain groups:

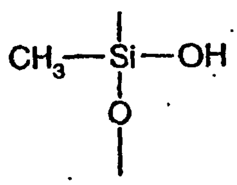

in proportions not exceeding 15% of the sum t+u+v.

**7.** Emulsion according to Claim 2, in which the polyorganosiloxanes comprising substituted or unsubstituted amine groups are chosen from:

(a) the polysiloxanes corresponding to the formula:

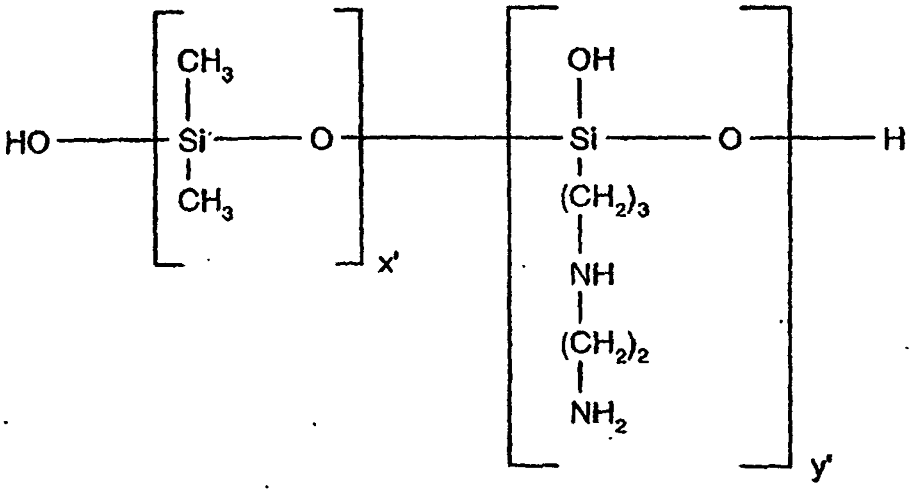

(VI)

in which x' and y' are integers such that the number-average molecular weight is between 5000 and 500,000;
(b) the cationic silicone polymers corresponding to the formula:

$$R^8{}_iG_{3-i}\text{-}Si(OSiG_2)_k\text{-}(OSiG_jR^8{}_{2-j})_l\text{-}O\text{-}SiG_{3-i}\text{-}R^8{}_i \qquad (VII)$$

in which:

G is a hydrogen atom or a phenyl, OH or $C_1$-$C_8$ alkyl group, for example methyl,
i denotes the number 0 or an integer from 1 to 3, in particular 0,
j denotes 0 or 1 and in particular 1,
k and l are numbers such that the sum (k+l) may range especially from 1 to 2000 and in particular from 50 to 150, n can denote a number from 0 to 1999 and especially from 49 to 149 and m can denote a number from 1 to 2000 and especially from 1 to 10;
$R^8$ is a monovalent radical of formula $-C_qH_{2q}L$ in which q is a number from 2 to 8 and L is an optionally quaternized amine group chosen from the groups:

$-NR^9-CH_2-CH_2-N(R^9)_2$

$-N(R^9)_2$

$-N^{\oplus}(R^9)_3A^-$

$-N(R^9)-CH_2-CH_2-N^{\oplus}R^9H_2\ A^-$,

in which $R^9$ denotes hydrogen, phenyl, benzyl or a saturated monovalent hydrocarbon-based radical, for example an alkyl radical containing from 1 to 20 carbon atoms and $A^-$ represents a halide ion;
(c) the cationic silicone polymers corresponding to the formula:

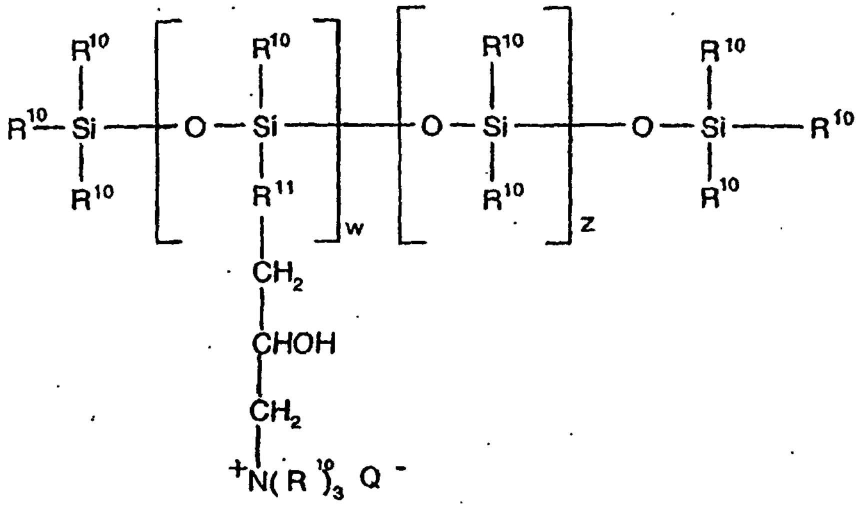

(VIII)

in which:

$R^{10}$ represents a monovalent hydrocarbon-based radical containing from 1 to 18 carbon atoms, and in particular a $C_1$-$C_{18}$ alkyl or $C_2$-$C_{18}$ alkenyl radical, for example methyl;
$R^{11}$ represents a divalent hydrocarbon-based radical, and in particular a $C_1$-$C_{18}$ alkylene radical or a divalent $C_1$-$C_{18}$, for example $C_1$-$C_8$, alkylenoxy radical; $Q^-$ is a halide ion;
w represents an average statistical value from 2 to 20 and in particular from 2 to 8;
z represents an average statistical value from 20 to 200 and in particular from 20 to 50.

**8.** Emulsion according to any one of Claims 1 to 7, in which the organomodified silicones are present in concentrations ranging from 0.1% to 40% relative to the total weight of the composition and more preferably in an amount ranging from 0.5% to 20%.

**9.** Emulsion according to any one of Claims 1 to 8, **characterized in that** it is provided in the form of a water-in-silicone emulsion.

**10.** Emulsion according to Claim 9, in which the organomodified silicone is chosen from those comprising oxyalkylenated groups as defined in Claim 2 or 3.

**11.** Emulsion according to any one of Claims 1 to 10, according to which the said sorghum extract is present in an amount which is effective for obtaining, 30 minutes after application to a fair skin at a rate of 2 $mg/cm^2$, a darkening **characterized in** the (L*, a*, b*) colorimetric measuring system by a $\Delta L^*$ ranging from -0.5 to -20.

**12.** Emulsion according to Claims 11, in which $\Delta L^*$ ranges from -0.5 to -15.

**13.** Emulsion according to any one of Claims 1 to 12, in which the said sorghum extract is present in an amount which is effective for obtaining, 30 minutes after application to a fair skin at a rate of 2 $mg/cm^2$, a coloration defined in the (L*, a*, b*) colorimetric measuring system by a ratio $\Delta a^*/\Delta b^*$ ranging from 0.5 to 3.

**14.** Emulsion according to Claim 13, in which the ratio $\Delta a^*/\Delta b^*$ ranges from 0.8 to 2.

**15.** Emulsion according to any one of Claims 1 to 14, in which the said sorghum extract is obtained from the whole plant, the stems, the seeds or the leaves of the genus Sorghum, in the fresh or dry state.

**16.** Emulsion according to Claim 15, in which the species of Sorghum are chosen from Sorghum bicolor, Sorghum caudatum, Sorghum nervosum, Sorghum durra, Sorghum vulgare and the Sorghum species in association with colletotrichum graminicola.

**17.** Emulsion according to any one of Claims 1 to 16, in which the said sorghum extract is obtained from the whole

plant, the stems, the seeds or the leaves of sorghum vulgare.

**18.** Composition according to any one of Claims 1 to 17, in which the sorghum extract may be obtained according to a process comprising the following steps:

(a) an extraction of parts from the whole plant, the stems, the seeds or the leaves of Sorghum in an aqueous medium which may also contain at least one organic solvent;
(b) a maceration in an alkaline medium having a pH of the order of 11-12;
(c) optionally a precipitation from the maceration medium by addition of an acid of the kind to reach a pH of the order of 1-2.

**19.** Emulsion according to any one of Claims 1 to 18, in which the concentration of sorghum extract in accordance with the invention ranges from 0.0001 to 10%, and preferably from 0.001 to 5% by weight, relative to the total weight of the composition.

**20.** Composition according to any one of Claims 1 to 19, additionally containing at least one mono- or polycarbonyl-containing self-tanning agent.

**21.** Composition according to Claim 20, in which the mono- or polycarbonyl-containing self-tanning agent(s) are chosen from isatin, alloxan, ninhydrin, glyceraldehyde, mesotartaric aldehyde, glutaraldehyde, erythrulose, pyrazoline-4,5-dione derivatives, dihydroxyacetone (DHA), 4,4-dihydroxypyrazoline-5-one derivatives, it being possible for these self-tanning agents to be combined or not with direct dyes or indole derivatives.

**22.** Composition according to Claim 21, in which the self-tanning agent is dihydroxyacetone (DHA).

**23.** Composition according to any one of Claims 20 to 22, in which the self-tanning agent is present in proportions ranging from 0.1 to 10% by weight relative to the total weight of the composition.

**24.** Composition according to any one of Claims 1 to 23, additionally containing at lest one UV radiation screening agent.

**25.** Composition according to Claim 24, in which the said UV radiation screening agent is chosen from organic UV screening agents or inorganic UV radiation screening agents.

**26.** Composition according to Claim 25, in which the organic UV screening agent(s) are water-soluble, fat-soluble or insoluble in the customary cosmetic solvents.

**27.** Composition according to Claim 26, in which the organic UV screening agent(s) are chosen from anthranilates; cinnamic derivatives; dibenzoylmethane derivatives; salicylic derivatives; camphor derivatives; triazine derivatives; benzophenone derivatives; β,β'-diphenylacrylate derivatives; benzotriazole derivatives; benzimidazole derivatives; imidazolines; bis-benzoazolyl derivatives; p-aminobenzoic acid (PABA) derivatives; methylenebis(hydroxyphenylbenzotriazole) derivatives; screening polymers and screening silicones; dimers derived from α-alkylstyrene; 4,4-diarylbutadiene derivatives and mixtures thereof.

**28.** Composition according to Claim 27, in which the organic UV radiation screening agent(s) are chosen from:

- Ethylhexyl Salicylate,
- Butyl Methoxydibenzoylmethane,
- Ethylhexyl methoxycinnamate,
- Octocrylene,
- Phenylbenzimidazole Sulfonic Acid,
- Terephthalylidene Dicamphor Sulfonic,
- Benzophenone-3,
- Benzophenone-4,
- Benzophenone-5,
- 4-Methylbenzylidene Camphor,
- Benzimidazilate,
- Anisotriazine,
- Ethylhexyl Triazone,

- Diethylhexyl Butamido Triazone,
- 2,4,6-Tris(diisobutyl 4'-aminobenzalmalonate)-s-triazine,
- Methylenebis-Benzotriazolyl Tetramethylbutylphenol,
- Drometrizole Trisiloxane,
- 1,1-Dicarboxy(2,2'-dimethyl-propyl)-4,4-diphenylbutadiene

and mixtures thereof.

**29.** Composition according to Claim 25, in which the inorganic UV radiation screening agent(s) are chosen from pigments or nanopigments of metal oxides which are coated or noncoated.

**30.** Composition according to Claim 29, in which the inorganic UV radiation screening agent(s) are chosen from nanopigments of titanium, iron, zinc, zirconium or cerium oxide, which are coated or uncoated.

**31.** Composition according to any one of Claims 24 to 30, in which the UV radiation screening agent(s) are present in proportions ranging from 0.1 to 15% by weight relative to the total weight of the composition.

**32.** Process for artificially colouring the skin close to that of a natural tan, **characterized in that** is consists in applying to the skin an effective amount of an emulsion as defined in any one of Claims 1 to 31.

**33.** Use of at least one sorghum extract and of at least one organomodified silicone as defined in the preceding claims in a cosmetic emulsion not containing a flavylium salt which is unsubstituted in position 3 and which is substituted with at least one hydroxyl or alkoxy radical, with the aim of giving the skin an artificial coloration close to that of a natural tan.